# EUROPEAN PATENT APPLICATION

(11) **EP 3 808 848 A1**
(43) Date of publication of application: **21.04.2021**
(21) Application number: 19203093.0
(22) Date of filing: 14.10.2019
(51) Int. Cl.: C12N 15/82, C12N 15/63, C12N 15/67, C12N 9/88, A01H 5/00

(54) **REGULATORY ELEMENT OF AN ARGININE DECARBOXYLASE GENE AND METHODS AND USES THEREOF**

(71) Applicant: Eberhard Karls Universität Tübingen, 72074 Tübingen (DE)
(72) Inventor: LAHAYE, Thomas, 72116 Mössingen (DE); WU, Dousheng, 410006 Changsha, Hunan (CN)
(74) Representative: Kuttenkeuler, David

(57) **Abstract**

The invention is based on the finding of a 5' translational regulation element in the *arginine decarboxylase* (*ADC*) gene (the ADC box). Modulation of the function of the ADC box results in a modulated protein expression which in turn has an effect on the polyamine content of a plant or plant cell. Hence the invention provides the ADC box, and mutated variants thereof, for the targeted modulation of gene expression. Further provided are methods, compounds and plants having modulated polyamine content. In addition the various methods and compounds of the invention have application in the modulation of the immune response of plants.

## Description

### FIELD OF THE INVENTION

The invention is based on the finding of a 5' translational regulation element in the *arginine decarboxylase (ADC)* gene (the ADC box) of land plants. Modulation of the function of the ADC box results in a modulated protein expression which in turn has an effect on the polyamine content of a plant or plant cell. Hence the invention provides the ADC box, and mutated variants thereof, for the targeted modulation of gene expression. Further provided are methods, compounds and plants having modulated polyamine content. In addition the various methods and compounds of the invention have application in the modulation of the immune response of plants.

### DESCRIPTION

Virulence of bacterial pathogens of plants and animals relies on effector proteins that bacteria inject via their type III (T3), type IV (T4), or type VI (T6) secretion systems directly into the cytoplasm of eukaryotic and prokaryotic target cells (Galán and Waksman, 2018; Garcia-Bayona and Comstock, 2018; Granato et al., 2019; Rüter et al., 2018). T4 and T6 effectors are typically injected into antagonistic bacteria where they act as potent toxins (Ma et al., 2014; Souza et al., 2015). By contrast, T3 effectors are generally injected into eukaryotic host cells where they manipulate cellular processes to promote bacterial growth (Büttner, 2016). It has been postulated that certain T3 effectors reprogram host cells to inhibit growth of microbial niche competitors (Rovenich et al., 2014). The lack of experimental evidence for such microbiota-influencing T3 effectors is possibly a consequence of testing environments that assess bacterial virulence solely in the context of binary host-microbe interactions, but not in the context of the postulated ternary microbe-host-microbe interactions.

TALEs typically bind to and transcriptionally activate 5-20 plant genes, with one of these genes, termed the host susceptibility (*S*) gene, contributing to bacterial virulence (Boch and Bonas, 2010; Boch et al., 2014). TALEs bind DNA via 15-30 tandem-arranged, near-identical 33-35 amino acid repeats. DNA base preference of TALE repeats is determined by the repeat-variable di-residue (RVD) located in repeat positions 12 and 13. The TALE code describes the one-to-one correspondence of RVD identities and their DNA base preference and facilitates prediction of TALE target sequences present in host promoters, commonly referred to as the effector binding element (*EBE*).

Differential transcriptome profiling of plant tissue infected with isogenic *Xanthomonas* strains containing or lacking a given TALE has been used in conjunction with *EBE* prediction to identify TALE-activated host S genes from numerous host plants (Boch et al., 2014). TALE-activated host *S* genes were found to encode sugar- and sulfate-transporters, transcription factors, and a pectate lyase (Boch et al., 2014; El Kasmi et al., 2018), and thus are functionally highly diverse. While the disease-promoting contribution of these *S* genes has been experimentally demonstrated, further studies are needed to clarify how elevated expression of plant S proteins promotes bacterial virulence.

*R.solanacearum* RipTALs contain 35 amino-acid, tandem-arranged repeats that are related to but distinct from *Xanthomonas* TALE repeats (de Lange et al., 2013; Schandry et al., 2016). Given the homology of *R.solanacearum* RipTALs to *Xanthomonas* TALEs, it is conceivable that RipTALs promote disease through transcriptional activation of host S genes.

Previously, a 17-bp sequence was identified that mediates *in planta* Brg11-dependent promoter-reporter activation (de Lange et al., 2013). However, no host promoters containing a Brg11-compatible target site (*Brg11-EBE*) have been identified, and thus the Brg11-induced host S gene that promotes bacterial disease remains to be elucidated.

Biogenic amines (BAs) are nitrogen compounds mainly produced by decarboxylation of amino acids, amination of aldehyde and ketone, and transamination. Such biogenic amines are low molecular weight compounds, which are synthesized during metabolism of microorganisms, plants, and animals, and thus are known as components easily found in cells thereof. Especially, polyamines such as spermidine, spermine, putrescine (or 1,4-butanediamine), cadaverine, etc., are substances present in most living cells.

Among them, putrescine is an important raw material which synthesizes polyamine nylon-4,6 by reacting with adipic acid, and is produced mainly by chemical synthesis through acrylonitrile and succinonitrile from propylene. In addition, a method for producing putrescine at high concentration by transformation of *E. coli* and genus *Corynebacterium* has been disclosed (International Publication No. WO06/005603; International Publication No. WO09/125924; Qian ZD et al., Biotechnol. Bioeng. 104(4): 651-662, 2009; Schneider et al., Appl. Microbiol. Biotechnol. 88(4): 859-868, 2010; and Schneider et al., Appl. Microbiol. Biotechnol. 95: 169-178., 2012). Further, research on putrescine transporter regarding *E. coli,* yeast, plant and animal cells has been actively conducted (K Igarashi, Plant Physiol. Biochem. 48: 506-512, 2010).

Thus, it is an objection of the invention to provide improved means for the production of polyamines, the application of TALE protein mediated immune control in plants as well as methods, uses and products applying Brg11 dependent gene regulation in a wider context of expression control and modulation.

### BRIEF DESCRIPTION OF THE INVENTION

Generally, and by way of brief description, the main aspects of the present invention can be described as follows:

In **a first aspect,** the invention pertains to a method for modulating the translation of a target messenger RNA (mRNA), the method comprising mutating at least one nucleotide in a genetic element (an ADC-box or a fragment thereof) contained in the 5' untranslated region encoding said mRNA, wherein said genetic element comprises in successive order GC rich regions: GC2 which is encoded by the sequence CCG(A/G)GGC; and GC3 which is encoded by the sequence GCCTCGG.

In **a second aspect,** the invention pertains to a method for modulating the expression of a protein coding gene, the method comprising introducing into the 5'untranslated region of the transcribed region of the protein coding gene an ADC-box, or a fragment thereof, to obtain a ADC-box modified protein coding gene, wherein said ADC-box, or a fragment thereof, comprises in successive (5' to 3') order GC rich regions GC2 which comprises the sequence CCG(A/G)GGC and GC3 which comprises the sequence GCCTCGG; and preferably wherein the ADC box is introduced between the transcriptional start and translational start of said 5' untranslated region.

In **a third aspect,** the invention pertains to an isolated nucleic acid comprising an ADC-box, or a fragment thereof, wherein said nucleic acid comprises an ADC-box sequence, or a fragment thereof, comprising in successive (5' to 3') order GC rich regions GC2 which comprises the sequence CCG(A/G)GGC and GC3 which comprises the sequence GCCTCGG.

In **a fourth aspect,** the invention pertains a system for the modulation of protein expression, the system comprising (i) a translation attenuation component which is an isolated nucleic acid according to the third aspect, and (ii) a transcriptional start modification component which is a nucleic acid for the expression of a TALE protein, or a TALE protein; and preferably wherein the TALE protein is Brg11.

In **a fifth aspect,** the invention pertains to a method for modulating protein coding gene expression in a cell, the method comprising the steps of
(i) Introducing an ADC-box, or a functional fragment thereof, into the 5' untranslated region of a target protein coding gene in a target cell in order to attenuate translation of mRNA expressed from said target protein coding gene, preferably wherein the ADC-box is introduced between the transcriptional start and the translational start of said 5' untranslated region;
(ii) Optionally, introducing into said target cell a TALE protein in order to initiate a transcriptional start 3' to the ADC-box and thereby generate an mRNA devoid of the ADC-box.

In **a sixth aspect,** the invention pertains to a method for increasing ADC activity in a cell, the method comprising introducing into the cell Brg11, or a variant thereof, or performing a method according to the first aspect.

In **a seventh aspect,** the invention pertains to a method for producing a plant with increased ADC activity, the method comprising modulating the expression of an ADC mRNA by a method according to the first aspect.

In **an eights aspect,** the invention pertains to a transgenic plant with modulated ADC gene expression, the transgenic plant comprising an ADC gene comprising a mutated ADC-box as recited in the first aspect.

In **a ninth aspect,** the invention pertains to a vector construct, comprising a nucleic acid according the previous aspects.

In **a tenth aspect,** the invention pertains to a recombinant host cell, comprising a nucleic acid, or a vector construct according to the previous aspects.

In **an eleventh aspect,** the invention pertains to a transgenic plant comprising a nucleic acid, a vector construct, or a recombinant host cell according to the previous aspects.

In **a twelfth aspect,** the invention pertains to a method for modulating an immune response of a plant or plant cell against pathogens which are sensitive to a polyamine mediated plant immune response, the method comprising the steps of a method of the sixth aspect.

In **a thirteenth aspect,** the invention pertains to a method for modulating gene expression, the method comprising
- Providing a target gene having in its 5' untranslated region, preferably between the transcriptional start and translational start, a translation regulatory element the presence of which in the transcribed mRNA results in a modulated translation of said mRNA, and
- Contacting the target gene with a TALE protein and thereby inducing a transcriptional start of the target gene in 3' direction of the regulatory element within the 5' untranslated region.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a method for modulating the translation of a target messenger RNA (mRNA), the method comprising mutating at least one nucleotide in a genetic element (an ADC-box or a fragment thereof) contained in the 5' untranslated region encoding said mRNA, wherein said genetic element comprises in successive order GC rich regions: GC2 which is encoded by the sequence CCG(A/G)GGC; and GC3 which is encoded by the sequence GCCTCGG.

The term "translation of a target mRNA" refers to the cell-biological process of ribosomal protein synthesis using mRNA as a template for amino acid carrying tRNAs according to the genetic code.

In preferred embodiments of the invention an mRNA is encoded in the genomic DNA, preferably in the form of a gene. Any mRNA comprises a stretch of complementary sequence to the gene it is transcribed from, and certain sequence elements, such as a poly-A tail, which are attached to the mRNA subsequent to or while transcription occurs. Additional processing steps include mRNA splicing, which removes non-coding intronic sequences. In context of the invention however, a region encoding an mRNA shall refer to a genomic location which provides the complementary sequence of a mature mRNA and from which said mRNA is transcribed (produced). Hence, in context of the present invention a "5' (prime) untranslated region encoding said mRNA" shall denote a genomic location in 5 prime direction of the translational start of a gene from which said target mRNA is transcribed. Usually such 5 prime untranslated region within genes comprises regulatory sequences that control RNA transcription. More generally, the term "untranslated region" as used herein refers to either of two sections, one on each side of a coding sequence on a strand of mRNA. If it is found on the 5' side, it is called the 5'UTR (or leader sequence), or if it is found on the 3' side, it is called the 3'UTR (or trailer sequence). The 5' UTR is upstream (in 5' direction) from the coding sequence as mentioned above. Within the 5' UTR is a sequence that is recognized by the ribosome which allows the ribosome to bind and initiate translation.

The term "nucleotide sequence" is intended to mean a continuous sequence of two or more nucleotides. The nucleotide may be DNA, RNA as well as a mixture thereof, and of natural, semi-synthetic or synthetic origin.

The term "coding sequence" or in short "cds" is intended to mean a nucleotide sequence which begins with, and includes, a start codon or first codon of the mature protein and ends with, and includes, a stop codon.

The term "genetic element" means any molecular unit which is or comprises a regulatory element. However, preferably a genetic element of the invention does not contain or comprise a sequence expressed as protein. A "regulatory sequence" of the invention refers to a nucleotide sequence located (preferably) upstream (5' non-coding sequences), within, or downstream (3' non-coding sequences) of a coding sequence, and which influences the transcription, RNA processing or stability, or translation of the associated coding sequence. The genetic element of the invention is preferably an *arginine decarboxylase* (*ADC*) box (the ADC box), or its derivatives as described herein.

The term "modification" as used herein, refers to the introduction of mutations into a nucleotide sequence, for example to substitutions, insertions or deletions of nucleic acid residues at specific positions in a nucleic acid sequence. The term "mutation" refers to the substitution, addition, deletion or replacement of single or multiple, non-consecutive or consecutive, nucleotides. The term "nucleic acid molecule" or "nucleic acid" is intended to indicate any single- or double stranded nucleic acid molecule of cDNA, genomic DNA, synthetic DNA or RNA, Peptide nucleic acid (PNA) or LNA origin.

The term "modulating" in context of the present invention shall denote preferably an increase in the level of translation of said target mRNA, and/or an increase in level of protein translated from said target mRNA.

The term "GC rich region" in context of the invention refers to a sequence stretch comprising more than 50% of G/C nucleotides. In particular the invention pertains to four GC rich regions which are abbreviated herein as GC1, GC2, GC3 and GC4. GC1 is encoded (on DNA level) by the sequence GGGGG(T/A); GC2 is encoded by the sequence CCG(A/G)GGC; GC3 is encoded by the sequence GCCTCGG; and GC4 is encoded by the sequence (G/-)CCCC(C/T). Genetic elements comprising the GC regions of the invention usually may form when transcribed as RNA a three-dimensional structure that is inhibitory to the translation machinery. In such elements GC2 pair (via complementary binding) with GC3, and/or GC1 interacts (complementary binds) with GC4. In the above sequences a slash indicates an "or", for example A/T refers to adenine or thymine on the respective position. Further the indicator "-" refers to no nucleotide.

In some preferred embodiments of the invention said genetic element comprises in successive order GC2 and GC3, more preferably the genetic element comprises in successive order four GC rich regions GC1, GC2, GC3 and GC4. The term "in successive order" in context of the invention shall mean the GC regions one after another in 3 prime or, preferably, 5 prime direction on DNA level. Between any two GC regions spacing nucleotides may exist.

In context of the various aspects and embodiments of the invention, a genetic element is preferred wherein GC1 and GC2 are separated by two nucleotides, preferably AG, such that the genetic element comprises a sequence encoded by: GGGGG(T/A)AGCCG(A/G)GGC (or GGGGG(U/A)AGCCG(A/G)GGC as mRNA - SEQ ID NO: 3), and preferably is GGGGGTAGCCGGGGC (SEQ ID NO: 4).

The genetic element of the invention preferably has an overall length of not more than 100 nucleotides, and preferably has a length of 40 to 80 nucleotides, and more preferably between 50 and 60 nucleotides. Moreover, in preferred embodiments the genetic element is located within the 5' untranslated region of an *arginine decarboxylase* (*ADC*) gene, and wherein said (target) mRNA is an mRNA of an *ADC.* Preferably, said at least one nucleotide is mutated within the genomic DNA encoding said target mRNA and thereby resulting in a mutated target mRNA, or is mutated in the transcribed mRNA.

The term "genomic" in context of the present invention shall pertain to all DNA sequence encoding for RNA, in particular the target mRNA of the invention. Such DNA sequence may be located within the genome of a cell, or outside of a genome, ectopically, for example within a plasmid, or mitochondrial, chloroplast, or any other transcribable DNA within a biological cell.

The method according to the first aspect comprises a step of mutating at least one nucleotide within the genetic element. Preferably, the at least one nucleotide to be mutated is located within any one of, or a combination of, GC1 to GC4. In some particular embodiments, mutating at least one nucleotide comprises the removal or deletion of at least one of GC1 to GC4, preferably comprises removal or deletion of at least 2 or 3 (such as GC2 and/or GC3), and most preferably removal or deletion of all four GC1 to GC4 regions. In other preferred embodiments of the invention mutating at least one nucleotide comprises mutating, preferably deleting, one nucleotide selected from nucleotides 5 to 10, most preferably deleting nucleotides 7 to 10; or 5,6 and 9, 10; or wherein mutating at least one nucleotide comprises inserting a nucleotide, for example inserting a thymine or adenine between nucleotides 6 and 7. The numbering refers to the nucleotide position as provided in the above definition of GC1 to GC4.

In context of the present invention mutations can be introduced by any means known to the skilled artisan. As a preferred embodiment the mutations would be created by classical methods of mutagenesis using random mutagenic irradiation or mutation inducing chemicals such as EMS; or biologically mutagenic entities such as transposable elements. However an alternative possibility is the use of gene editing approaches. As used herein the term "gene editing" or genome editing refers to a type of genetic engineering in which DNA is inserted, replaced, or removed from a genome using artificially engineered nucleases, or "molecular scissors". The nucleases create specific double-strand breaks (DSBs) at desired locations in the genome, and harness the cell's endogenous mechanisms to repair the induced break by natural processes of homologous recombination (HR) and nonhomologous end-joining (NHEJ). The general mechanism and recent advances of CRISPR system is discussed in Cong, L. et al., "Multiplex genome engineering using CRISPR systems," Science, 339(6121): 819-823 (2013); Fu, Y. et al., "High-frequency off-target mutagenesis induced by CRISPR-Cas nucleases in human cells," Nature Biotechnology, 31, 822-826 (2013); Chu, VT et al. "Increasing the efficiency of homology- directed repair for CRISPR-Cas9-induced precise gene editing in mammalian cells," Nature Biotechnology 33, 543-548 (2015); Shmakov, S. et al, "Discovery and functional characterization of diverse Class 2 CRISPR-Cas systems," Molecular Cell, 60, 1-13 (2015); Makarova, KS et al, "An updated evolutionary classification of CRISPR-Cas systems,", Nature Reviews Microbiology, 13, 1- 15 (2015). Site-specific cleavage of a target DNA occurs at locations determined by both 1) base- pairing complementarity between the guide RNA and the target DNA (also called a protospacer) and 2) a short motif in the target DNA referred to as the protospacer adjacent motif (PAM). For example, an engineered cell can be generated using a CRISPR system, e.g., a type II CRISPR system. A Cas enzyme used in the methods disclosed herein can be Cas9, which catalyzes DNA cleavage.

In some preferred embodiments, the methods and plants of the invention do not include, or are not exclusively obtained by, an essentially biological process, such as the crossing and selecting of while genomes.

Methods described herein preferably take advantage of a CRISPR system. Therefore, in a preferred embodiment the genetic element of the invention is mutated preferably by a component of a CRISPR mediated gene editing approach, and most preferably is a guide or sgRNA specific for the genetic element, or a GC box therein). There are at least five types of CRISPR systems which all incorporate RNAs and Cas proteins. Types I, III, and IV assemble a multi-Cas protein complex that is capable of cleaving nucleic acids that are complementary to the crRNA. Types I and III both require pre-crRNA processing prior to assembling the processed crRNA into the multi-Cas protein complex. Types II and V CRISPR systems comprise a single Cas protein complexed with at least one guiding RNA.

Enzymatic action by Cas9 derived from *Streptococcus pyogenes* or any closely related Cas9 can generate double stranded breaks at target site sequences which hybridize to 20 nucleotides of a guide sequence and that have a protospacer-adjacent motif (PAM) following the 20 nucleotides of the target sequence.

A vector can be operably linked to an enzyme-coding sequence encoding a CRISPR enzyme, such as a Cas protein (CRISPR- associated protein). Non-limiting examples of Cas proteins can include Casl, CaslB, Cas2, Cas3, Cas4, Cass, Cas6, Cas7, Cas8, Cas9 (also known as Csnl or Csxl2), CaslO, Csyl, Csy2, Csy3, Csel, Cse2, Cscl, Csc2, Csas, Csn2, Csm2, Csm3, Csm4, Csm5, Csm6, Cmrl, Cmr3, Cmr4, Cmrs, Cmr6, Csbl, Csb2, Csb3, Csxl7, Csxl4, CsxlO, Csxl6, CsaX, Csx3, Csxl, CsxlS, Csfl, Csf2, CsO, Csf4, Cpfl, C2cl, C2C3, Cas9HiFi, homologues thereof, or modified versions thereof. An unmodified CRISPR enzyme can have DNA cleavage activity, such as Cas9. A CRISPR enzyme can direct cleavage of one or both strands at a target sequence, such as within a target sequence and/or within a complement of a target sequence. For example, a CRISPR enzyme can direct cleavage of one or both strands within or within about 1, 2, 3, 4, 5, 6, 7, 8, 9, 10, 15, 20, 25, 50, 100, 200, 500, or more base pairs from the first or last nucleotide of a target sequence. A vector that encodes a CRISPR enzyme that is mutated with respect to a corresponding wild-type enzyme such that the mutated CRISPR enzyme lacks the ability to cleave one or both strands of a target polynucleotide containing a target sequence can be used. A Cas protein can be a high fidelity cas protein such as Cas9HiFi.

A vector that encodes a CRISPR enzyme comprising one or more nuclear localization sequences (NLSs), such as more than or more than about 1, 2, 3, 4, 5, 6, 7, 8, 9, 10, NLSs can be used. For example, a CRISPR enzyme can comprise more than or more than about 1, 2, 3, 4, 5, 6, 7, 8, 9, 10, NLSs at or near the ammo-terminus, more than or more than about 1, 2, 3, 4, 5, 6, 7, 8, 9, 10, NLSs at or near the carboxyl-terminus, or any combination of these (e.g. , one or more NLS at the ammo- terminus and one or more NLS at the carboxyl terminus). When more than one NLS is present, each can be selected independently of others, such that a single NLS can be present in more than one copy and/or in combination with one or more other NLSs present in one or more copies.

Cas9 can refer to a polypeptide with at least or at least about 50%, 60%, 70%, 80%, 90%, 100% sequence identity and/or sequence similarity to a wild type exemplary Cas9 polypeptide (e.g., Cas9 from S. pyogenes). Cas9 can refer to a polypeptide with at most or at most about 50%, 60%, 70%, 80%, 90%, 100% sequence identity and/or sequence similarity to a wild type exemplary Cas9 polypeptide (e.g. , from S. pyogenes). Cas9 can refer to the wild type or a modified form of the Cas9 protein that can comprise an amino acid change such as a deletion, insertion, substitution, variant, mutation, fusion, chimera, or any combination thereof.

A polynucleotide encoding an endonuclease (e.g., a Cas protein such as Cas9) can be codon optimized for expression in particular cells, such as eukaryotic cells. This type of optimization can entail the mutation of foreign-derived (e.g., recombinant) DNA to mimic the codon preferences of the intended host organism or cell while encoding the same protein.

An endonuclease can comprise an amino acid sequence having at least or at least about 50%, 60%, 70%, 75%, 80%, 85%, 90%, 95%, 99%, or 100%, amino acid sequence identity to the nuclease domain of a wild type exemplary site-directed polypeptide (e.g., Cas9 from S. *pyogenes*)*.* While *S. pyogenes* Cas9 (SpCas9), is commonly used as a CRISPR endonuclease for genome engineering, it may not be the best endonuclease for every target excision site. For example, the PAM sequence for SpCas9 (5' NGG 3') is abundant throughout the human genome, but a NGG sequence may not be positioned correctly to target a desired gene for modification. In some cases, a different endonuclease may be used to target certain genomic targets. In some cases, synthetic SpCas9-derived variants with non-NGG PAM sequences may be used. Additionally, other Cas9 orthologues from various species have been identified and these "non-SpCas9s" bind a variety of PAM sequences that could also be useful for the present invention. For example, the relatively large size of SpCas9 (approximately 4Mb coding sequence) means that plasmids carrying the SpCas9 cDNA may not be efficiently expressed in a cell. Conversely, the coding sequence for Staphylococcus aureus Cas9 (SaCas9) is approximately 1 kilo base shorter than SpCas9, possibly allowing it to be efficiently expressed in a cell. Similar to SpCas9, the SaCas9 endonuclease is capable of modifying target genes in mammalian cells in vitro and in mice in vivo. Alternatives to *S. pyogenes* Cas9 may include RNA-guided endonucleases from the Cpfl family that display cleavage activity in mammalian cells. Unlike Cas9 nucleases, the result of Cpf 1-mediated DNA cleavage is a double-strand break with a short 3' overhang. Cpfl 's staggered cleavage pattern may open up the possibility of directional gene transfer, analogous to traditional restriction enzyme cloning, which may increase the efficiency of gene editing. Like the Cas9 variants and orthologues described above, Cpfl may also expand the number of sites that can be targeted by CRISPR to AT- rich regions or AT-rich genomes that lack the NGG PAM sites favored by SpCas9.

As used herein, the terms "guide RNA (gRNA)" and "single guide RNA (sgRNA)", and their grammatical equivalents can refer to an RNA which can be specific for a target DNA and can form a complex with a Cas protein. A guide RNA can comprise a guide sequence, or spacer sequence, that specifies a target site and guides an RNA/Cas complex to a specified target DNA for cleavage. Site-specific cleavage of a target DNA occurs at locations determined by both 1) base-pairing complementarity between a guide RNA and a target DNA (also called a protospacer) and 2) a short motif in a target DNA referred to as a protospacer adjacent motif (PAM).

In **a second aspect,** the present invention provides a method for modulating the expression of a protein coding gene, the method comprising introducing into the 5'untranslated region of the transcribed region of the protein coding gene an ADC-box, or a fragment thereof, to obtain a ADC-box modified protein coding gene, wherein said ADC-box, or a fragment thereof, comprises in successive (5' to 3') order GC rich regions GC2 which comprises the sequence CCG(A/G)GGC and GC3 which comprises the sequence GCCTCGG; and preferably wherein the ADC box is introduced between the transcriptional start and translational start of said 5' untranslated region. In this aspect, the genetic element, and the GC boxes therein, are as defined for the first aspect of the invention. Similarly, the above definitions apply equally to the other aspects of the invention.

Preferably, modulating the expression of a protein coding gene is a decrease in the level of protein translation of the mRNA transcribed from said protein coding gene, and/or a decrease in the level of protein translated from the mRNA transcribed from said protein coding gene, and/or is an increase of mRNA decay of the mRNA transcribed from said protein coding gene.

A preferred embodiment of the second aspect then comprises a further step of expressing said ADC-box modified protein coding gene in a cell.

The method according to the second aspect, in preferred embodiments, further comprises a step of contacting the ADC-box modified protein coding gene in said cell with a bacterial transcription-activator-like effector (TALE) protein, such as preferably Brg11 (*Ralstonia solanacearum*), or a variant thereof. Brg11 in context of the present invention shall refer to a protein with an amino acid sequence according to SEQ ID NO: 1. A variant thereof is a protein having at least 50%, 60%, 70%, 80%, 90%, 95%, 96%, 97%, 98%, 99% sequence identity in its amino acid sequence to the sequence shown in SEQ ID NO: 1. A variant is preferably a functional equivalent of the protein shown in SEQ ID NO: 1.

In a **third aspect** the invention pertains to an isolated nucleic acid comprising an ADC-box, or a fragment thereof, wherein said nucleic acid comprises an ADC-box sequence, or a fragment thereof, comprising in successive (5' to 3') order GC rich regions GC2 which comprises the sequence CCG(A/G)GGC and GC3 which comprises the sequence GCCTCGG. The isolated nucleic acid according in particular embodiments is preferred, wherein said genetic element comprises in successive order four GC rich regions GC1, GC2, GC3 and GC4, and wherein GC1 is encoded by the sequence GGGGG(T/A); and GC4 is encoded by the sequence (G/-)CCCC(C/T).

The definitions of the ADC box, GC1 to GC4 and genetic element are as described herein for the other aspects and embodiments of the invention. The term "isolated nucleic acid" refers to the nucleic acid fragment that is separated from the naturally associated nucleic acid in its natural source, which includes DNA/RNA fragments obtained by artificial gene synthesis or cloning approaches, in particular fragments created by PCR.

In preferred embodiments of the invention the isolated nucleic acid comprises a protein coding gene, and wherein said functional ADC-box said sequence, or a fragment thereof, is located or placed within the 5' untranslated region of said protein coding gene. Hence, in preferred embodiments the invention pertains to an isolated and artificial nucleic acid construct comprising the 5 prime to 3 prime order the ADC box or genetic element according to the invention, a transcriptional start, a translational start followed by the protein coding sequence of a gene to be expressed.

Further preferred according to the invention is an isolated nucleic acid, wherein the protein coding gene encodes for an ADC, and wherein the ADC-box sequence, or a fragment thereof, is non-functional and/or mutated. An ADC according to the invention is preferably any *arginine decarboxylase* (*ADC*) such as the protein shown in SEQ ID NO: 2, the ADC of *Solanum lycopersicum* (Tomato). The term ADC shall include all homologs, orthologs or paralogs of said protein. In addition or alternatively, the ADC of the invention is a protein having an amino acid sequence with at least 50%, 60%, 70%, 80%, 90%, 95%, 96%, 97%, 98%, 99% sequence identity to the sequence shown in SEQ ID NO: 2, and preferably having arginine decarboxylase function.

In alternative embodiments the protein coding gene encodes for a protein other than an ADC, and wherein the ADC-box sequence, or a fragment thereof, is functional, therefore allows for the binding of a TALE protein such as Brg11, which binding induces an alternative transcript. In such embodiments the functional ADC box sequence is a sequence when expressed as RNA attenuates translation of said RNA, for example by forming an inhibitory three-dimensional structure in the mRNA transcript. Such attenuated translation is impaired by binding of a TALE such as Brg11 by inducing a shorter transcript lacking the respective sequence forming the three dimensional structure responsible for translation attenuation.

Preferably, the ADC-box sequence, or fragment thereof, has an overall length of not more than 100 nucleotides, and preferably has a length of 40 to 80 nucleotides, and more preferably between 50 and 60 nucleotides.

In a **fourth aspect** there is provided a system for the modulation of protein expression, the system comprising (i) a translation attenuation component which is an isolated nucleic acid according to the previous aspects, and (ii) a transcriptional start modification component which is nucleic acid for the expression of a TALE protein, or a TALE protein; and preferably wherein the TALE protein is Brg11.

As used herein, the term "TALE (transcription activator-like effector)" refers to a protein secreted by pathogenic bacteria when they infect various plant cells. The protein can bind promoter sequences in the host plant and activate the expression of plant genes that aid bacterial infection or inhibit the expression of plant genes that interfere with the infection, or indeed support immune reactions against other pathogens (Current Opinion in Plant Biology, vol. 13, pp. 394-401, August 2010; Science, vol. 326, pp. 1501, Dec. 11 2009. The term "TAL effector," and "TALE" refer to a class of highly specific DNA binding proteins that harbor highly conserved repeat domains that each bind to a single base pair of DNA. The identities of two residues (referred to as repeat variable di-residues or RVDs) in these 33 to 35 amino acid repeats are associated with the binding specificity of these domains. Three assembly platforms have been described for achieving sequence-specific TAL effector proteins that may be suitably employed in the TAL effector proteins described herein. Those assembly platforms include: (1) solid-phase methods; (2) standard cloning methods; and (3) Golden Gate assembly methods. The solid phase assembly of DNA fragments encoding TAL effector repeat arrays using multichannel pipets or automated liquid handling robots is described in Reyon et al., Nat. Biotechnol. 30:460-465 (2012); Briggs et al., Nucleic Acids Res. 40(15):e117 (2012); and Wang et al., Angew Chem. Int. Ed. Engl. 51(34):8505-8508 (2012). The REAL methodology for the hierarchical assembly of DNA fragments encoding TAL effector repeat arrays using standard restriction digestion and ligation cloning methods is described in Sander et al., Nat. Biotechnol. (2011) and Huang et al. Nat. Biotechnol. (2011). "REAL-Fast" is a faster version of REAL, which follows the same assembly protocol as REAL but utilizes plasmids encoding pre-assembled TAL repeats rather than single TAL repeats. See, Reyon et al., Curr Protoc Mol Biol. (2012). "Golden Gate" methods for assembling DNA encoding TAL effector repeat arrays, which methods are based on the simultaneous ligation of multiple DNA fragments encoding TAL repeat domains, are described by Cermak et al., Nucleic Acids Res. (2011); Li et al., Nucleic Acids Res. (2011); Morbitzer et al., Nucleic Acids Res. (2011); Weber et al., PLoS One (2011); Zhang et al., Nat. Biotechnol. (2011); and Li et al., Plant Mol. Biol. (2012). The crystal structure of a TAL effector (PthXol) bound to its DNA target site has recently been determined. Mak et al., Science 335(6069):716-9 2012; e-pub 5 Jan. 2012 PubMed PMID: 22223736. These crystal structure data permit the precise definition of the boundaries of DNA recognition region and facilitates strategies for the creation of well-behaved TALE fusion constructs, which may be applied to achieve highly sequence specific nucleotide sequence detection. Specifically-designed TAL effector proteins can be tested for activity against a cognate target site and for off-target activity against any closely related genomic targets.

In **a fifth aspect,** the invention pertains to a method for modulating protein coding gene expression in a cell, the method comprising the steps of
(iii) Introducing an ADC-box, or a functional fragment thereof, into the 5' untranslated region of a target protein coding gene in a target cell in order to attenuate translation of mRNA expressed from said target protein coding gene, preferably wherein the ADC-box is introduced between the transcriptional start and translational start of said 5' untranslated region;
(iv) Optionally, introducing into said target cell a TALE protein in order to initiate a transcriptional start 3' to the ADC-box and thereby generate an mRNA devoid of the ADC-box.

In **a sixth aspect,** the invention pertains to a method for increasing ADC activity in a cell, the method comprising introducing into the cell Brg11, or a variant thereof, or preforming a method according to the first aspect.

In context of the present invention the term "ADC activity" is an enzymatic conversion of arginine to agmatine.

The method of the sixth aspect is particular useful for the modulation of the polyamine metabolism in the cell, preferably is for increasing the level of polyamines such as putrescine in the cell.

A cell preferably used in such methods is a eukaryotic cell, such as an animal or plant cell.

The ADC used in an ADC as described elsewhere herein. Preferably the method is for increasing ADC activity in a plant, more preferably for increasing in a plant any one or a combination of the following:
- Number of leaves;
- Number of flowers;
- Root area,:
- higher area to perimeter ratio of leaves.

In **a seventh aspect,** the invention pertains to a method for producing a plant with increased ADC activity, the method comprising modulating the expression of an ADC mRNA by a method according to the first aspect.

In **an eights aspect,** the invention pertains to a transgenic plant with modulated ADC gene expression, the transgenic plant comprising an ADC gene comprising a mutated ADC-box as recited in the first aspect.

In **a ninth aspect,** the invention pertains to a vector construct, comprising a nucleic acid according the previous aspects.

In **a tenth aspect,** the invention pertains to a recombinant host cell, comprising a nucleic acid, or a vector construct according to the previous aspects.

In **an eleventh aspect,** the invention pertains to a transgenic plant comprising a nucleic acid, a vector construct, or a recombinant host cell according to the previous aspects.

In **a twelfth aspect,** the invention pertains to a method for modulating an immune response of a plant or plant cell against polyamine-sensitive pathogens, the method comprising the steps of a method of the sixth aspect.

In **a thirteenth aspect,** the invention pertains to a method for modulating gene expression, the method comprising
- Providing a target gene having in its 5' untranslated region, preferably between the transcriptional start and translational start, a translation regulatory element the presence of which in the transcribed mRNA results in a modulated translation of said mRNA, and
- Contacting the target gene with a TALE protein and thereby induce a transcriptional start of the target gene in 3' direction of the regulatory element within the 5' untranslated region.

A translation regulatory element is preferably a genetic element or an ADC-box as defined in any one of the preceding aspects.

The method according to the present aspect is preferred, wherein the step (ii) results in a shorter length of the mRNA 5'UTR transcribed from the target gene.

In further preferred embodiments said translation regulatory element comprises a TALE protein binding site, and a translation inhibitory element, wherein the translation inhibitory element when present in the expressed mRNA reduces translation of said mRNA compared to the same mRNA not comprising the translation inhibitory element.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1: A method for modulating the translation of a target messenger RNA (mRNA), the method comprising mutating at least one nucleotide in a genetic element (an ADC-box or a fragment thereof) contained in the 5' untranslated region encoding said mRNA, wherein said genetic element comprises in successive order GC rich regions GC2 which is encoded by the sequence CCG-A/G-GGC and GC3 which is encoded by the sequence GCCTCGG.
Item 2: The method according to item 2, wherein said genetic element comprises in successive order four GC rich regions GC1, GC2, GC3 and GC4, and wherein GC1 is encoded by the sequence GGGGG-T/A; and GC4 is encoded by the sequence G/o-CCCC-C/T.
Item 3: The method according to item 1 or 2, wherein the at least one nucleotide is located within any one of GC1 to GC4.
Item 4: The method according to any one of items 1 to 3, wherein the genetic element has an overall length of not more than 100 nucleotides, and preferably has a length of 40 to 80 nucleotides, and more preferably between 50 and 60 nucleotides.
Item 5: The method according to any one of items 1 to 4, wherein the genetic element is within the 5' untranslated region of an *arginine decarboxylase (ADC)* gene, and wherein said mRNA is an mRNA of an *ADC.*
Item 6:The method according to any one of items 1 to 5, wherein the modulating is an increase in the level of translation of said target mRNA, and/or an increase in level of protein translated from said target mRNA.
Item 7: The method according to any one of items 1 to 6, wherein said at least one nucleotide is mutated in the genomic DNA encoding said target mRNA and thereby resulting in a mutated target mRNA, or is mutated in the transcribed mRNA.
Item 8: The method according to any one of items 1 to 7, wherein mutating at least one nucleotide comprises the removal or deletion of at least one of GC1 to GC4, preferably comprises removal or deletion of at least 2 or 3, and most preferably removal or deletion of all four GC1 to GC4 regions.
Item 9: The method according to any one of items 1 to 7, wherein GC1 and GC2 are separated by two nucleotides, preferably AG, such that the genetic element comprises a sequence encoded by: GGGGG(T/A)AGCCG(A/G)GGC (or GGGGG(U/A)AGCCG(A/G)GGC as mRNA), and preferably is GGGGGTAGCCGGGGC.
Item 10: The method according to item 9, wherein mutating at least one nucleotide comprises mutating, preferably deleting, one nucleotide selected from nucleotides 5 to 10, most preferably deleting nucleotides 7 to 10; or 5,6 and 9, 10; or wherein mutating at least one nucleotide comprises inserting a nucleotide, for example inserting a thymine or adenine between nucleotides 6 and 7.
Item 11: The method according to any one of items 1 to 11, wherein said mutating at least one nucleotide is done by gene editing
Item 12: A method for modulating the expression of a protein coding gene, the method comprising introducing into the 5'untranslated region of the transcribed region of the protein coding gene an ADC-box, or a fragment thereof, to obtain a ADC-box modified protein coding gene, wherein said ADC-box, or a fragment thereof, comprises in successive (5' to 3') order GC rich regions GC2 which comprises the sequence CCG-A/G-GGC and GC3 which comprises the sequence GCCTCGG; and preferably wherein the ADC box is introduced between the transcriptional start and translational start of said 5' untranslated region.
Item 13: The method according to item 12, wherein said genetic element comprises in successive order four GC rich regions GC1, GC2, GC3 and GC4, and wherein GC1 is encoded by the sequence GGGGG-T/A; and GC4 is encoded by the sequence G/o-CCCC-C/T.
Item 14: The method according to item 12 or 13, wherein the ADC-box or the fragment thereof, has an overall length of not more than 100 nucleotides, and preferably has a length of 40 to 80 nucleotides, and more preferably between 50 and 60 nucleotides.
Item 15: The method according to any one of items 12 to 14, wherein modulating the expression of a protein coding gene is a decrease in the level of protein translation of the mRNA transcribed from said protein coding gene, and/or a decrease in the level of protein translated from the mRNA transcribed from said protein coding gene, and/or is an increase of mRNA decay of the mRNA transcribed from said protein coding gene.
Item 16: The method according to any one of items 13, and 14 to 15 when referring to item 13, wherein GC1 and GC2 are separated by two nucleotides, preferably AG, such that the genetic element comprises a sequence encoded by: GGGGG(T/A)AGCCG(A/G)GGC (or GGGGG(U/A)AGCCG(/A/G)GGC as mRNA), and preferably is GGGGGTAGCCGGGGC.
Item 17: The method according to any one of items 12 to 16, comprising a further step of expressing said ADC-box modified protein coding gene in a cell.
Item 18: The method according to item 17, further comprising a step of contacting the ADC-box modified protein coding gene in said cell with a bacterial transcription-activator-like effector (TALE) protein, such as preferably Brg11 (*Ralstonia solanacearum*)*,* or a variant thereof.
Item 19: An isolated nucleic acid comprising an ADC-box, or a fragment thereof, wherein said nucleic acid comprises an ADC-box sequence, or a fragment thereof, comprising in successive (5' to 3') order GC rich regions GC2 which comprises the sequence CCG-A/G-GGC and GC3 which comprises the sequence GCCTCGG.
Item 20: The isolated nucleic acid according to item 19, wherein said genetic element comprises in successive order four GC rich regions GC1, GC2, GC3 and GC4, and wherein GC1 is encoded by the sequence GGGGG-T/A; and GC4 is encoded by the sequence G/o-CCCC-C/T.
Item 21: The isolated nucleic acid according to item 17 or 18, which comprises a protein coding gene, and wherein said functional ADC-box sequence, or a fragment thereof, is located within the 5' untranslated region of said protein coding gene.
Item 22: The isolated nucleic acid according to item 21, wherein
   (i) the protein coding gene encodes for an ADC, and wherein the ADC-box sequence, or a fragment thereof, is non-functional and/or mutated.
   (ii) The protein coding gene encodes for a protein other than an ADC, and wherein the ADC-box sequence, or a fragment thereof, is functional.
Item 23: The isolated nucleic acid according to item 22, wherein the functional ADC box sequence is a sequence when expressed as RNA attenuates translation of said RNA.
Item 24: The isolated nucleic acid according to any one of items 19 to 23, wherein the ADC-box sequence, or fragment thereof, has an overall length of not more than 100 nucleotides, and preferably has a length of 40 to 80 nucleotides, and more preferably between 50 and 60 nucleotides.
Item 25: A system for the modulation of protein expression, the system comprising (i) a translation attenuation component which is an isolated nucleic acid according to any one of items 19 to 24, and (ii) a transcriptional start modification component which is nucleic acid for the expression of a TALE protein, or a TALE protein; and preferably wherein the TALE protein is Brg11.
Item 26: A method for modulating protein coding gene expression in a cell, the method comprising the steps of
   (i) Introducing an ADC-box, or a functional fragment thereof, into the 5' untranslated region of a target protein coding gene in a target cell in order to attenuate translation of mRNA expressed from said target protein coding gene, preferably wherein the ADC-box is introduced between the transcriptional start and translational start of said 5' untranslated region;
   (ii) Optionally, introducing into said target cell a TALE protein in order to initiate a transcriptional start 3' to the ADC-box and thereby generate an mRNA devoid of the ADC-box.
Item 27: A method for increasing ADC activity in a cell, the method comprising introducing into the cell Brg11, or a variant thereof, or preforming a method according to any one of items 1 to 11.
Item 28: The method according to item 27, wherein the ADC activity is an enzymatic conversion of arginine to agmatine.
Item 29: The method according to item 27 or 28, wherein the method is for the modulation of the polyamine metabolism in the cell, preferably is for increasing the level of polyamines such as putrescine in the cell.
Item 30: The method according to any one of items 24 to 26, wherein the cell is a eukaryotic cell, such as an animal or plant cell.
Item 31: The method according to any one of items 27 to 30, wherein the ADC is ADC1 or ADC2, a variant protein, or a homolog, paralog or ortholog thereof.
Item 32: The method according to any one of items 27 to 31, wherein the method is for increasing ADC activity in a plant.
Item 33: The method according to any one of items 27 to 32, wherein the method is for increasing in a plant any one or a combination of the following:
   (i) Number of leaves;
   (ii) Number of flowers;
   (iii) Root area,:
   (iv) higher area to perimeter ratio of leaves.
Item 34: A method for producing a plant with increased ADC activity, the method comprising modulating the expression of an ADC mRNA by a method according to any one of items 1 to 11.
Item 35: A transgenic plant with modulated ADC gene expression, the transgenic plant comprising an ADC gene comprising a mutated ADC-box as recited in any one of items 1 to 11.
Item 36: A vector construct, comprising a nucleic acid according to any one of items 19 to 24.
Item 37: A recombinant host cell, comprising a nucleic acid according to any one of items 19 to 24, or a vector construct according to item 36.
Item 38: A transgenic plant comprising a nucleic acid according to any one of items 19 to 24, a vector construct according to item 36, or a recombinant host cell according to item 37.
Item 39: A method for modulating an immune response of a plant or plant cell against polyamine-sensitive pathogens, the method comprising the steps of a method according to any one of items 27 to 32.
Item 40: A method for modulating gene expression, the method comprising
   (i) Providing a target gene having in its 5' untranslated region, preferably between the transcriptional start and translational start, a translation regulatory element the presence of which in the transcribed mRNA results in a modulated translation of said mRNA,
   (ii) Contacting the target gene with a TALE protein and thereby induce a transcriptional start of the target gene in 3' direction of the regulatory element within the 5' untranslated region.
Item 41: The method according to item 40, wherein the translation regulatory element is an genetic element or an ADC-box as defined in any one of the preceding items.
Item 42: The method according to item 40 or 41, wherein the step (ii) results in a shorter length of the mRNA 5'UTR transcribed from the target gene.
Item 43: The method according to any one of items 40 to 42, wherein said target gene is an artificially modified gene to contain said translation regulatory element.
Item 44: The method according to any one of items 40 to 43, wherein said translation regulatory element comprises a TALE protein binding site, and a translation inhibitory element, wherein the translation inhibitory element when present in the expressed mRNA reduces translation of said mRNA compared to the same mRNA not comprising the translation inhibitory element.

### BRIEF DESCRIPTION OF THE FIGURES AND SEQUENCES

The figures show:

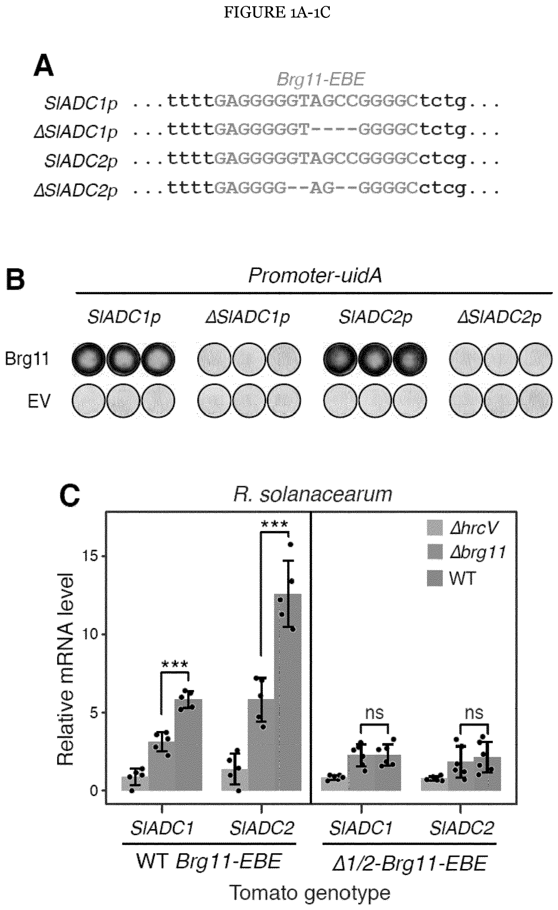

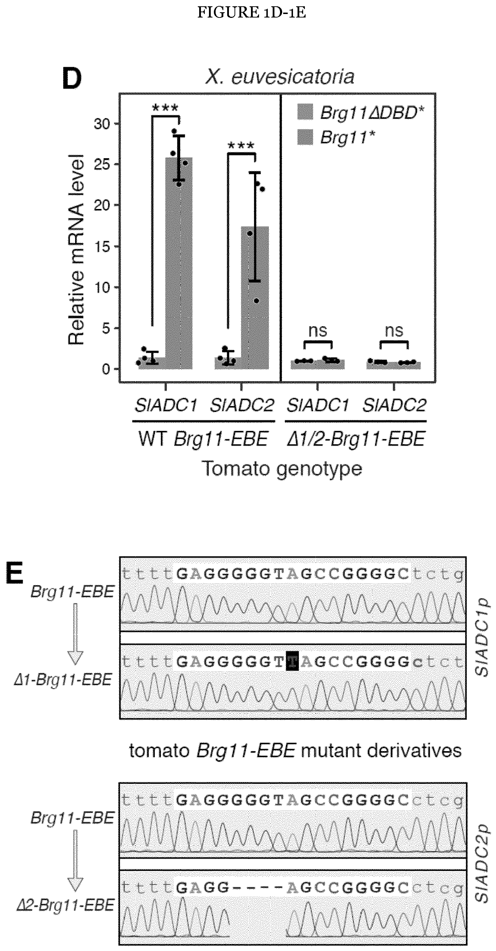

**Figure 1****:** shows that the *R. solanacearum* effector Brg11 transcriptionally activates tomato *ADC* Genes in a *Brg11-EBE* dependent manner; (A) Schematic display of promoter-reporter constructs studied in (B). Blue font indicates the *Brg11-EBE* and mutated derivatives (Δ) within the *SlADC1*/*2* promoters (*SlADC1*/*2p*). Hyphens indicate deleted nucleotides. (B) Gene activation by Brg11 depends on integrity of the *Brg11-EBE.* Depicted promoter-*uid*A fusions were co-delivered with either *35S* promoter-driven *brg11* (Brg11) or an empty vector control (EV) into *N. benthamiana* leaves via *A. tumefaciens.* GUS staining was carried out two days post infiltration. (C and D) Delivery of Brg11 into tomato leaves activates *SlADC1*/*2* genes in a *Brg11-EBE*-dependent manner. Height of the bars represents *SlADC1*/*2* mRNA levels in depicted tomato genotypes measured by qPCR upon inoculation of depicted *R. solanacearum* (C) and *X*. *euvesicatoria* (D) strains. Data are mean ± SD; ****P* < 0.001 (Student's t-test); ns, not significant; solid circles show values of individual biological samples. (E) CRISPR-Cas9 mediated mutations in the *Brg11-EBEs* of *SlADC1p* and *SlADC2p.* Mutations in *SlADC1* and *SIADC2* promoters are indicated by black background and hyphens, respectively.

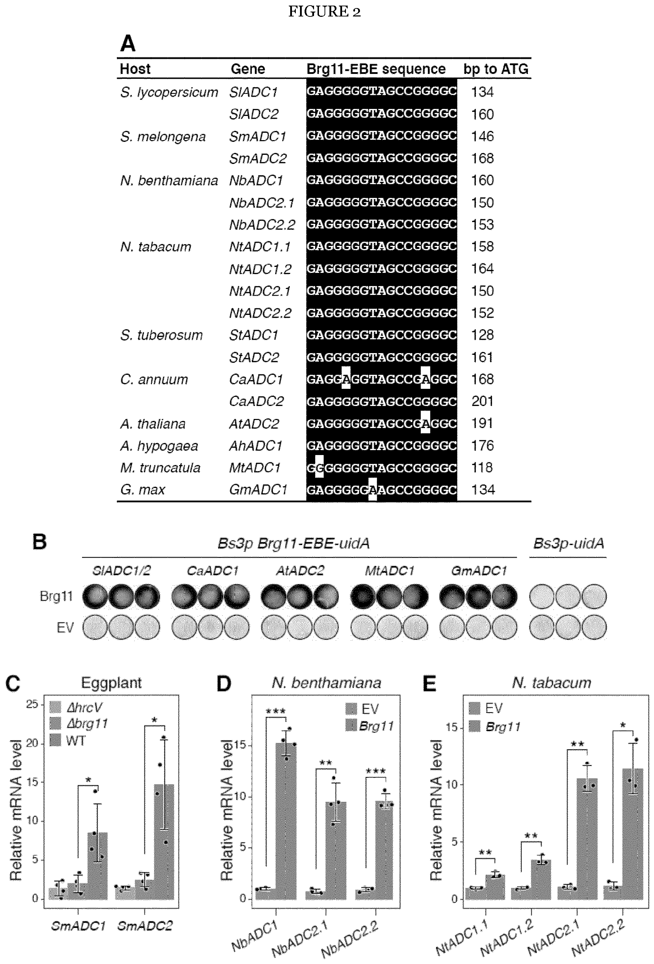

**Figure 2****:** shows that Brg11 targets *ADC* genes across different *R. solanacearum* hosts; (A) The *Brg11-EBE* is conserved across multiple *R. solanacearum* host species. Nucleotides of depicted host species that differ with respect to the *Brg11-EBE* in *SlADC1*/*2* (uppermost sequence) are highlighted by black font on white background. (B) Nucleotide polymorphisms in *Brg11-EBEs* of distinct host species do not affect Brg11-mediated gene activation. *UidA* reporter gene constructs, driven by *Bs3* promoter-derivatives containing the depicted *Brg11-EBEs,* were co-delivered with either a *35S* promoter-driven *Brg11* (Brg11) or empty vector control (EV) into *N. benthamiana* leaves via *A. tumefaciens.* GUS staining of leaf discs was carried out at two days post infiltration. (C-E) Brg11 induces increase of *ADC* transcripts in three representative host species. Plant leaves were infiltrated either with depicted *R. solanacearum* strains (C) or *A*. *tumefaciens* strains (D and E) containing either a *35S* promoter-driven *Brg11* or EV. Infiltrated leaves were harvested at one day (C) or two days (D and E) after inoculation for qPCR analysis. Data are mean ± SD (n = 4 for eggplant and *N. benthamiana,* n = 3 for *N. tabacum*)*; *P* < 0.05; ***P* < 0.01; ****P* < 0.001 (Student's t-test); solid circles, individual biological replicates.

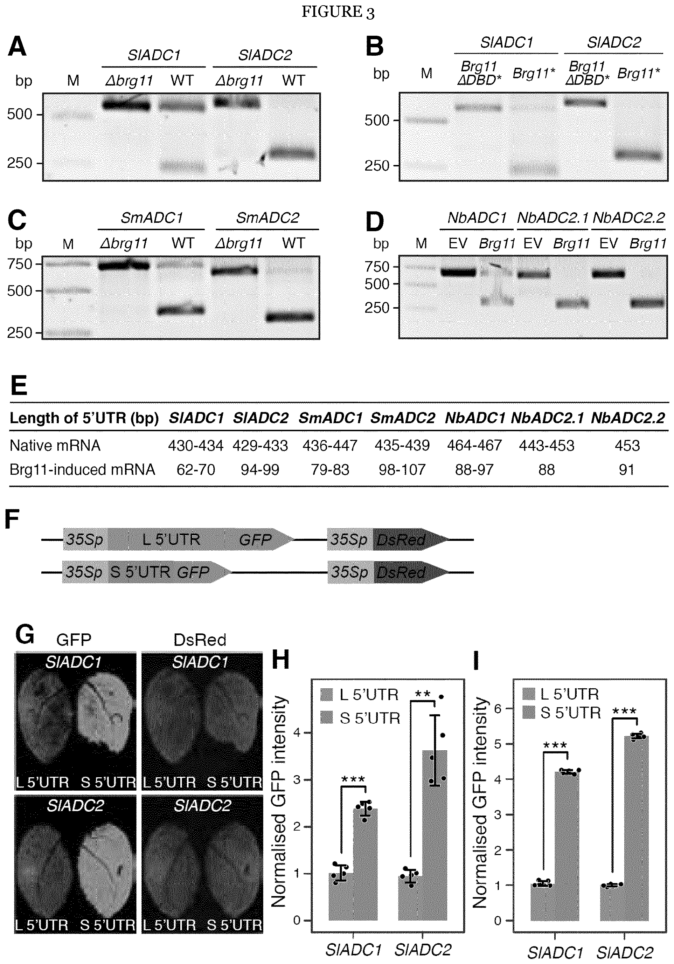

**Figure 3****:** shows that Brg11 induced *ADC* transcripts with short 5'UTRs have higher translational activity than native *ADC* transcripts with long 5'UTRs; (A-D) 5'RACE in three representative *R. solanacearum* hosts shows that Brg11 induces *ADC* transcripts with short 5'UTRs. 5'RACE was conducted on (A) tomato (*Solanum lycopersicum* [*Sl*]) inoculated with *R*. *solanacearum,* (B) tomato inoculated with *X. euvesicatoria,* (C) eggplant (*Solanum melongena* [*Sm*]) inoculated with *R. solanacearum* or (D) N. *benthamiana* (*Nb*) inoculated with *A. tumefaciens.* M, DNA marker. (E) Length of 5'UTRs of native and Brg11-induced *ADC* transcripts in three representative *R. solanacearum* host species. (F) Schematic display of the dual reporter constructs used to determine *in planta* translational activity of *SlADC1*/*2* 5'UTRs. *35S*-promoter driven *DsRed* was used for normalization. L 5'UTR, long 5'UTR from native *ADC* transcripts; S 5'UTR, short 5'UTR of Brg11-induced *ADC* transcripts. (G and H) The short 5'UTR of Brg11-induced *ADC* mRNAs and the long 5'UTR of native *ADC* mRNAs have high and low translational activity in *vivo,* respectively. 36 hours after *A. tumefaciens* mediated delivery of reporter constructs with depicted 5'UTRs into *N. benthamiana* leaves GFP fluorescence was monitored visually (G) and evaluated quantitatively (H). n = 5. Data are mean ± SD; ***P* < 0.01; ****P* < 0.001 (Student's t test); solid circles, individual biological replicates. (I) Brg11-induced short *SlADC1*/*2* 5'UTRs mediate high level of translation *in vitro.* Translation efficiency of either *SlADC1*/*2* L 5'UTR- or the S 5'UTR-*GFP* fusions were determined *in vitro* using tobacco BY2 cell-free lysates. GFP intensity was quantified 2 hours after addition of mRNA (n = 6). Data are mean ± SD; ****P* < 0.001 (Student's t-test); solid circles, individual biological replicates.

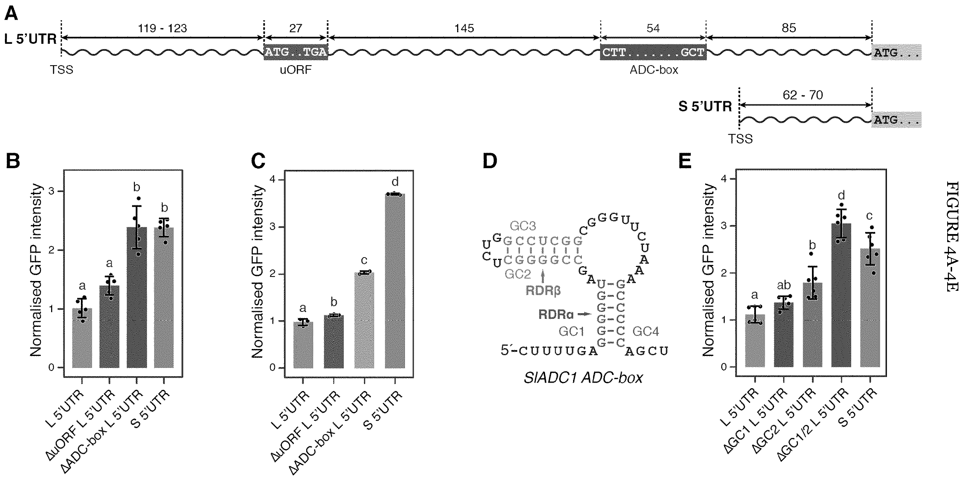

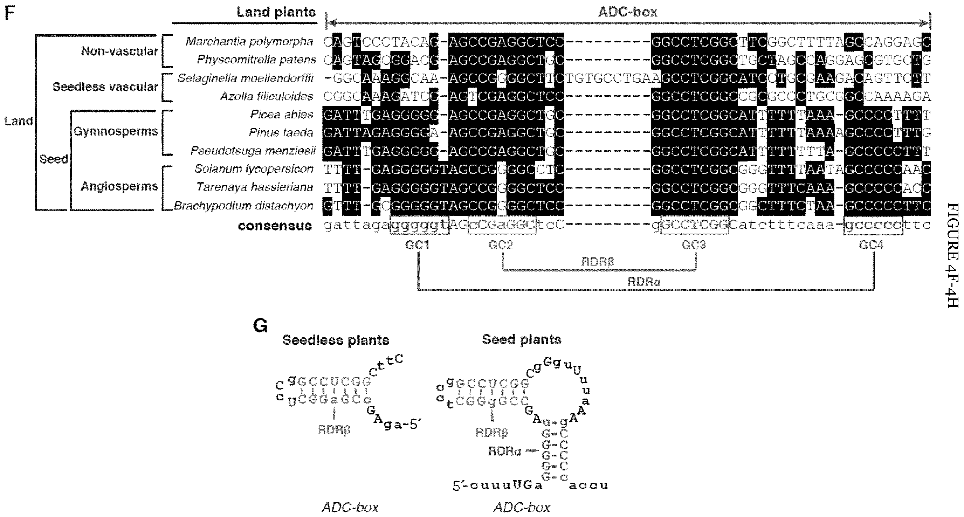

**Figure 4****:** shows that the *ADC-box* is conserved across land plant species and acts as a translational repressor; (A) Schematic display of the 5'UTRs of long native *SlADC1* transcripts (L 5' UTR) and short Brg11-induced *SlADC1* transcripts (S 5' UTR). Numbers indicate bp-distances between depicted 5'UTR elements. TSS, transcription start site. (B) Deletion of the *ADC-box* increases translation of corresponding 5'UTRs in *vivo.* Reporter constructs containing the L 5'UTR, the S 5'UTR or derivatives lacking either the uORF (ΔuORF) or the *ADC-box* (ΔADC-box) were delivered into *N. benthamiana* leaves via *A. tumefaciens.* GFP fluorescence was quantified at 36 hpi. n = 5. Data are mean ± SD; lowercase letters indicate significance in one-way analysis of variance (ANOVA, TukeyHSD); solid circles, individual biological replicates. (C) Deletion of the *ADC-box* but not the uORF from the long 5'UTR of native *SlADC1* transcripts causes increased reporter expression *in vitro. In vitro* translation activity of the *SlADC1* S 5'UTR, the *SlADC1* L 5'UTR, and corresponding uORF or *ADC-box* deletion derivates (ΔuORF and ΔADC-box, respectively) were determined 2 hours after addition of in *vitro*-transcribed reporter mRNAs (n = 3). Data are mean ± SD; lowercase letters indicate significance in one-way analysis of variance (ANOVA, TukeyHSD); solid circles, individual biological replicates. (D) Predicted RNA secondary structure of the *ADC-box.* GC-rich regions (GC1-4) and RNA duplex regions (RDRα [purple font], RDRβ [blue font]) are depicted. (E) The two predicted duplex regions in the 5'UTR of *SlADC1* have additive translational inhibitory effects. Reporter constructs containing the L 5'UTR, the S 5'UTR or corresponding derivatives with mutations in GC1 (ΔGC1), GC2 (ΔGC2) or both (ΔGC1/2) were delivered into *N. benthamiana* leaves using *A*. *tumefaciens.* GFP fluorescence was quantified at 36 hpi. n = 6. Data are mean ± SD; lowercase letters indicate significance in one-way analysis of variance (ANOVA, TukeyHSD); solid circles, individual biological replicates. (F) Seedless plants are conserved in GC2/3 but not in GC1/4. (G) Tentative RNA secondary structure formed by *ADC-boxes* of seedless plants and seed plants. Capital letter indicates highly conserved nucleotides.

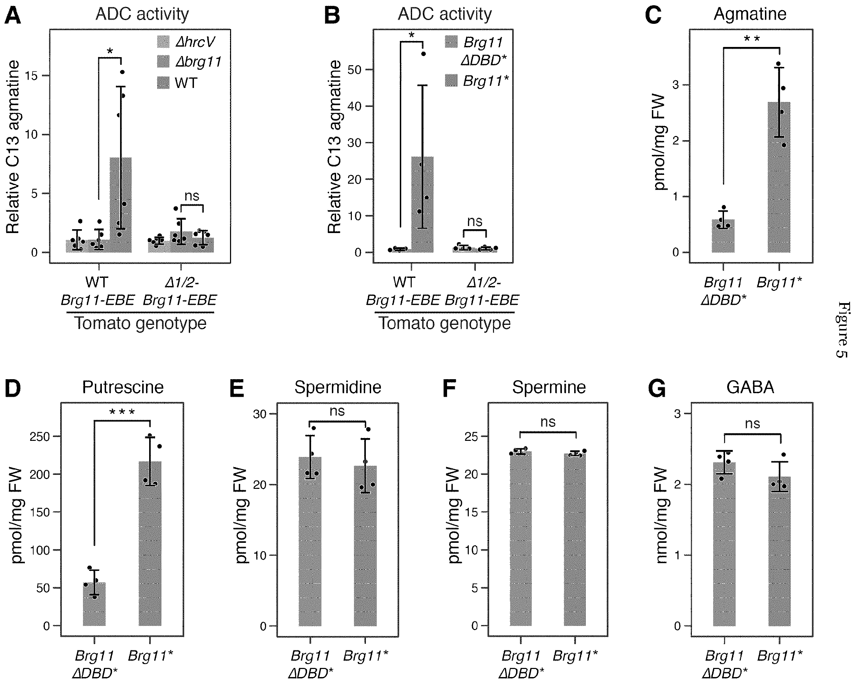

**Figure 5****:** shows that Brg11 increases ADC activity and putrescine levels; (A and B) Brg11 increases ADC activity in a *Brg11-EBE*-dependent manner in tomato. Tomato leaves were inoculated with depicted *R. solanacearum* (A) or *X. euvesicatoria* (B) strains and inoculated tissue was collected 18 h (A) or 24 h (B) after infiltration. ¹³C arginine was added to homogenized tissue of inoculated tomato leaves and one hour later ¹³C agmatine was quantified by mass spectrometry (MS) thus providing a proxy for ADC activity. n = 6 for (A) and n = 4 for (B). Data are mean ± SD; **P* < 0.05; ns, not significant (Student's t test); solid circles, individual biological replicates. (C-G) The depicted metabolites were quantified by MS, 24 hours after inoculation with depicted *X. euvesicatoria* strains in wild-type tomato. n = 4. Data are mean ± SD; ***P* < 0.01; ****P* < 0.001; ns, not significant (Student's t test); solid circles, individual biological replicates.

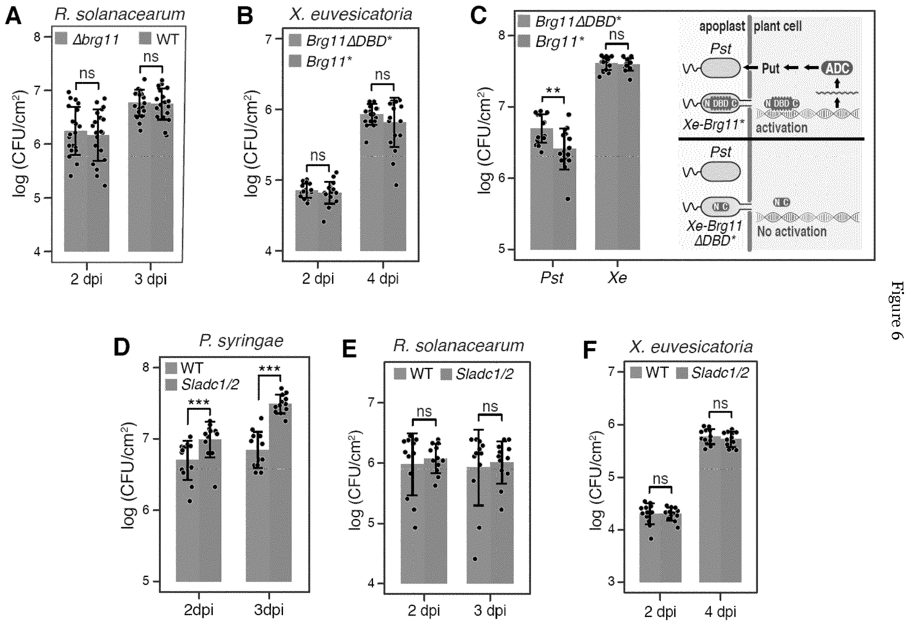

**Figure 6****:** shows that Brg11 inhibits *in planta* Growth of *P. syringae* but has no impact on *in planta* growth of *X*. *euvesicatoria* and *R. solanacearum;* (A and B) Brg11 has no impact on *in planta* growth of *R. solanacearum* and *X. euvesicatoria* in tomato. Bacterial counts of *R*. *solanacearum* (A) and *X. euvesicatoria* (B) were determined at indicated days post inoculation (dpi) upon infiltration with bacteria at 10⁵ CFU/ml (n = 20). Data are mean ± SD; ns, not significant (Student's t-test); solid circles, individual biological replicates. (C) *X. euvesicatoria* strain containing Brg11 reduces *in planta* growth of co-inoculated *P. syringae.* Bacterial populations in tomato wild-type plants were quantified upon mixed inoculation of *P. syringae* pv. *tomato* DC3000 (*Pst*) and *X. euvesicatoria* (*Xe*) delivering Brg11* or Brg11ΔDBD* at 10⁵ CFU/ml and 10⁸ CFU/ml, respectively, at 2 dpi (n = 15). The picture on the right explains the conceptual basis the mixed inoculation. Data are mean ± SD; ***P* < 0.01; ns, not significant (Student's t-test); solid circles, individual biological replicates. (D) *Sladc1*/*2* double mutants are more susceptible to *Pst* than tomato wild-type plants. Bacterial growth of inoculated tomato leaves was quantified at depicted timepoints. Inoculation was carried with depicted bacteria at 10⁵ CFU/ml (n = 12). Data are mean ± SD; ****P* < 0.001 (Student's t-test); solid circles, individual biological replicates. (E and F) *In planta* growth of *R. solanacearum* and *X*. *euvesicatoria* wild-type strains in tomato *Sladc1*/*2* double mutants (*Sladc1*/*2*) and tomato wild-type plants (WT) does not differ. Bacterial growth of inoculated tomato leaves (E and F) was quantified at depicted timepoints. Inoculation was carried with depicted bacteria at 10⁵ CFU/ml (n = 12). Data are mean ± SD; ns, not significant (Student's t-test); solid circles, individual biological replicates.

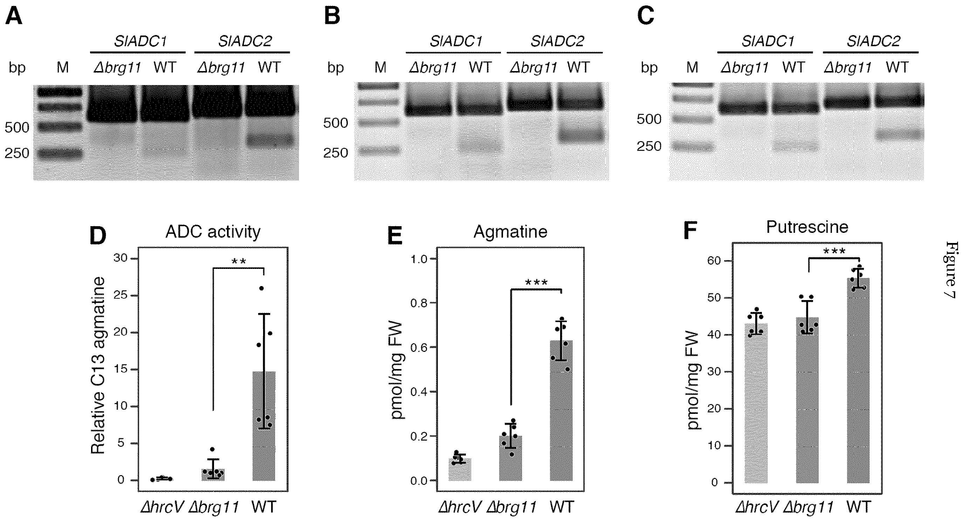

**Figure 7****:** shows that Brg11 is functional in tomato root; (A-C) 5'RACE PCR on tomato root tissue after inoculation with the *R. solanacearum* brg11 mutant strain (Δbrg11) and the wild-type strain (WT) on (A) Seven-day-old seedlings with root cut (B) one-week-old seedlings with vacuum infiltration and (C) four-week-old tomato plants with soil drenching. M, DNA marker. (D-F) Brg11 induces ADC activity, agmatine and putrescine level in tomato root. The depicted metabolites were quantified by MS, 48 hours after vacuum infiltration with depicted *R*. *solanacearum* strains in wild-type tomato. n = 6. Data are mean ± SD; ***P* < 0.01; ****P* < 0.001 (Student's t test); solid circles, individual biological replicates.

The sequences show:
**SEQ ID NOs. 1 shows the amino acid sequence of Brg11 (UniProt entry Q8XYE3):** 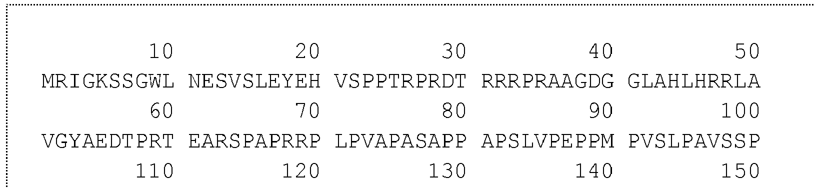 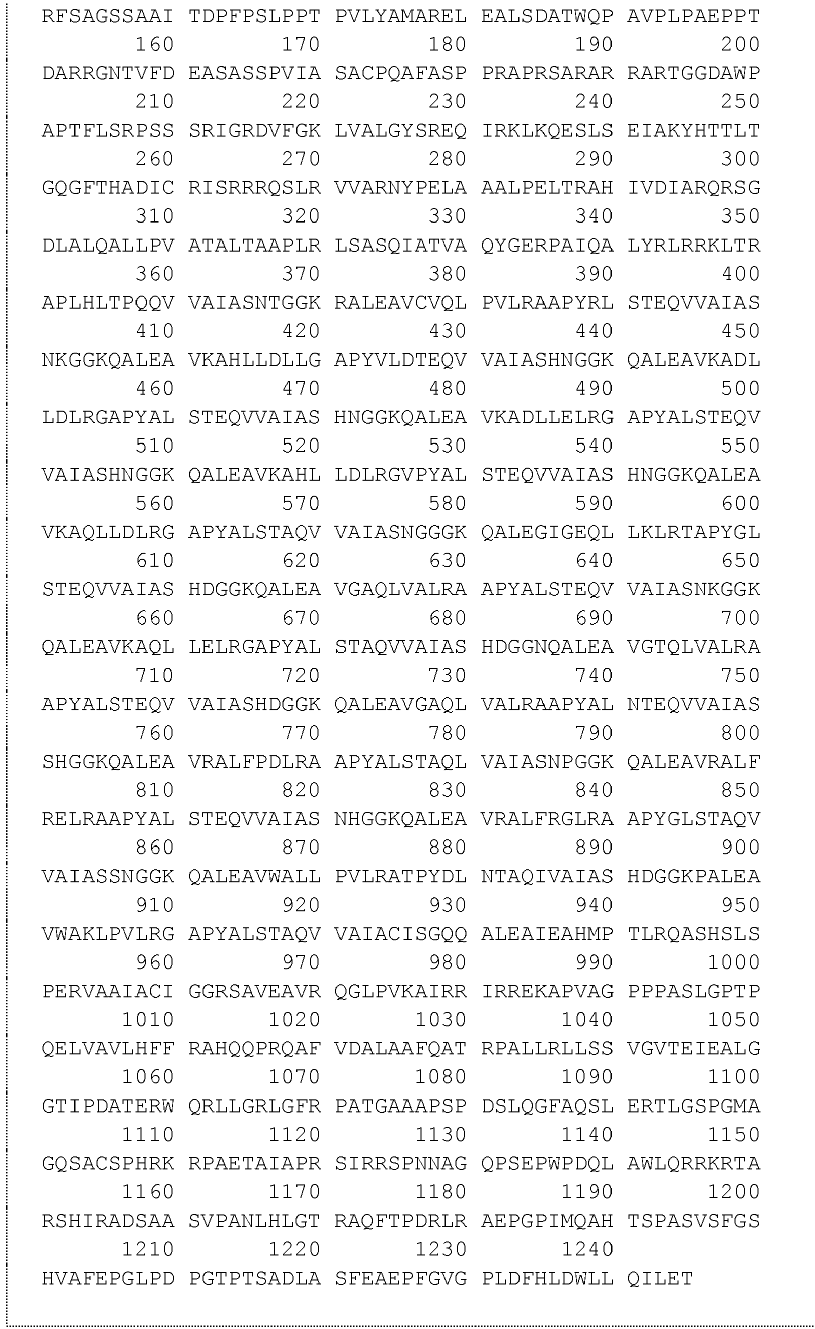
**SEQ ID NOs. 2 shows the amino acid sequence of ADC from tomato (UniProt entry EoADH6_SOLLC):** 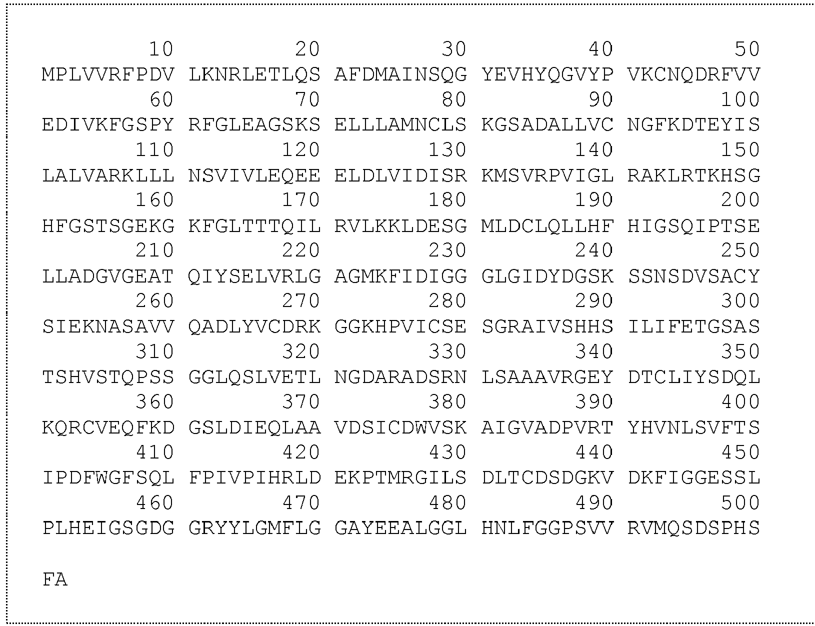
**SEQ ID NOs. 3-27 show the nucleic acid sequences as disclosed in the appended set of figures.**

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Brg11 targets exclusively host ADC genes

The Brg11 containing *R. solanacearum* strain GMI1000 was originally isolated from tomato (Salanoubat et al., 2002). Accordingly, the inventors scanned the tomato promoterome for sequences related to the previously identified 17 bp Brg11 target sequence to identify Brg11-activated host genes in tomato (de Lange et al., 2013). Each of the top 25 predicted *EBEs* was inserted into a minimal promoter and studied for Brg11-dependent activation using promoter-reporter fusions. These assays revealed that only the two sequence-identical *EBEs* upstream of *SlADC1* and *SlADC2* genes mediate Brg11-dependent reporter activation. To confirm functionality of these *Brg11-EBEs* in the context of their native promoters, *SlADC1*/*2* upstream sequences (∼350 bp 5' of ATG) were fused to a reporter gene and it was found that both sequences mediate Brg11-dependent reporter activation (Figures 1A and 1B). Notably, mutating the *Brg11-EBEs* in *SlADC1*/*2* upstream sequences abolished Brg11-dependent reporter activation (Figures 1A and 1B), demonstrating that the integrity of the *Brg11-EBEs* is crucial to Brg11-dependent gene activation.

To determine if Brg11 can activate genome-embedded *SlADC1*/*2* genes, *R. solanacearum* strains with or without Brg11 were delivered into tomato leaves via blunt-end syringe infiltration. The Brg11-containing strain was observed to increase *SlADC1*/*2* transcript levels approximately two-fold relative to the Brg11-lacking strain (Figure 1C). Since *R. solanacearum* is a root-adapted pathogen, it seemed plausible that T3 secretion system (T3SS)-mediated delivery of Brg11, and thus activation of *SlADC1*/*2* genes in leaf tissue, was moderate. Therefore *SlADC1*/*2* activation was studies following T3SS-mediated delivery of Brg11 via the tomato leaf pathogen *X. euvesicatoria.* To do so, the N-terminal translocation domain of an *X. euvesicatoria* effector was fused to either Brg11 or a Brg11-derivative lacking the DNA binding domain (Brg11ΔDBD) yielding Brg11* and Brg11ΔDBD*, respectively. It was observed that *X. euvesicatoria* T3SS-mediated delivery of Brg11* induced 15- to 25-fold higher *SlADC1*/*2* transcript levels relative to Brg11ΔDBD* (Figure 1D). To test if Brg11-dependent activation of *SlADC1*/*2* depends on the integrity of corresponding upstream *Brg11-EBEs,* heritable mutations were generated in both *Brg11-EBEs* by CRISPR-Cas9 mutagenesis (Figure 1E). Indeed, Brg11-dependent activation of *SlADC1*/*2* was abolished in the tomato double mutant lacking functional *Brg11-EBEs* upstream of *SlADC1*/*2 (Δ1*/*2-Brg11-EBE*) (Figures 1C and 1D). This demonstrates that Brg11 activates the transcription of *SlADC1*/*2* genes, and that this activation is reliant on the integrity of Brg11 target sites upstream of the *SlADC1*/*2* coding sequences (CDS).

The inventors then sought to clarify whether Brg11 has other direct target genes besides *SlADC1*/*2.* Using RNA-seq, tomato transcriptomes were compared 24 hours after inoculation with *X. euvesicatoria* strains containing either Brg11* or Brg11ΔDBD*. 117 host transcripts, including *SlADC1*/*2* showed a Brg11-dependent increase (fold-change > 2.0, corrected *p* < 0.05). In addition to *SlADC1*/*2, Sl12g009000* was the only induced tomato gene with a *Brg11-EBE-*like sequence upstream of its CDS. However, it was found that the putative *Brg11-EBE* upstream of *Sl12g00900* does not mediate Brg11-dependent reporter activation. Taken together, these data show that *SlADC1* and *SlADC2* are the only direct target genes of Brg11 in tomato.

The Brg11 containing *R. solanacearum* strain GMI1000 (alternative strain designations: UQRS 442; JS 753) was originally isolated from tomato (Prior and Steva, 1990; Salanoubat et al., 2002), but it infects a broad range of important agricultural crops such as eggplant, tobacco, potato, and pepper (Guidot et al., 2014). It was thus hypothesized that Brg11 activates *ADC* genes not only in tomato, but across many or possibly all *R. solanacearum* host species. In silico studies uncovered the *Brg11-EBE* sequence upstream of *ADC* genes in all ten investigated host genomes (Figure 2A). In four of the host species, however, the *Brg11-EBEs* had polymorphisms with respect to the tomato *Brg11-EBE.* Promoter-reporter assays showed however, that these polymorphisms do not disturb Brg11-dependent activation (Figure 2B). Next, Brg11 was delivered into three representative host species (*Solanum melongena* [eggplant], *Nicotiana benthamiana* and *Nicotiana tabacum*) to study if Brg11 activates these genome-embedded *ADC* genes. The inventors found Brg11-dependent *ADC* transcript accumulation in all three host species (Figures 2C-2E), suggesting that Brg11 targets *ADC* genes in most, if not all, *R. solanacearum* host plants.

### Example 2: Brg11 induces truncated, highly-active ADC mRNAs

ADCs are rate-limiting enzymes in polyamine (PA) biosynthesis (Alcazar and Tiburcio, 2014). The inventors performed 5' rapid amplification of cDNA ends (RACE) to determine the 5'UTR of *ADC* transcripts in the presence and absence of Brg11. It was found that the 5'UTR of Brg11-induced *ADC* transcripts in tomato, eggplant and *N. benthamiana* was consistently >300 bp shorter than the 5'UTR of corresponding native *ADC* transcripts (Figures 3A-3E).

To clarify if the long 5'UTR of native *ADC* transcripts and the short 5'UTR of Brg11-induced *ADC* transcripts differ in translational activity, the inventors cloned long and short 5'UTRs of *SlADC1*/*2* genes upstream of a *GFP* reporter under transcriptional control of the constitutive *35S* promoter (Figure 3F). The T-DNA contained in addition a constitutively expressed dsRED fluorophore for normalization of GFP reporter read out. Delivery of these reporter constructs into *N. benthamiana* leaves via *Agrobacterium* showed that constructs with the short 5'UTR had 2.5- to 3.5-fold higher GFP intensity relative to constructs containing the long 5'LTTR (Figures 3G and 3H). No significant differences were observed in reporter mRNA levels, suggesting that different 5'UTRs do not cause differences in mRNA stability. The inventors complemented these *in planta* assays with *in vitro* translation assays using tobacco cell lysates (Buntru et al., 2014; Buntru et al., 2015). In agreement with the *in planta* assays, the *in vitro* studies showed that constructs containing the short 5'UTR had approximately 4-fold higher GFP intensity relative to constructs containing the long 5'UTR (Figure 3I). Quantification of reporter mRNA levels after *in vitro* translation showed no significant differences in transcript levels. These results suggest that Brg11-induced host *ADC* transcripts with truncated 5'LTTRs have higher translational activity than native *ADC* transcripts.

### Example 3: An ancient cis-element limits translation of ADC mRNAs

The low translational activity observed for long 5'UTRs of native *SlADC1*/*2* transcripts suggests the presence of *cis-*elements that inhibit translation. In addition to small upstream ORFs (uORFs), GC-rich regions within the 5'UTR can repress translation since they lead to secondary RNA structures that impede ribosomal scanning for the initiation codon (Barrett et al., 2012; Wachter, 2014). The inventors identified a ∼50 bp GC-rich sequence motif in the 5'UTR of native *ADC* transcripts that is conserved in genomes of seed plant species (embryophyta [gymnosperms and angiosperms]) which was designated as the *"ADC-box".* Notably, the uORF and the transcribed *ADC-box* are both present in the long 5'UTR of native *SlADC1*/*2* transcripts, but absent from the short 5'UTR of Brg11-induced *SlADC1*/*2* transcripts (Figure 4A). To determine the functional relevance of the uORF and the *ADC-box,* the inventors generated corresponding *STADC1* deletion derivatives and tested their translational activity by the *in vivo* and *in vitro* assays described above. In both assays, deletion of the *ADC-box* significantly increased translational activity, and in contrast, deletion of the uORF only slightly increased translational activity (Figures 4B and 4C). These results suggest that the *ADC-box* has a more profound inhibitory effect on translation of *ADC* transcripts than the uORF. It is worth noting that native *ADC* transcripts from several plant species lack an uORF (e.g., *Poaceae* species), but they do contain an *ADC-box,* suggesting that uORFs but not the *ADC-box* are functionally dispensable in these *ADC* transcripts.

Based on the conserved angiosperm sequences, an RNA secondary structure was predicted for the *ADC-box* that utilizes four consecutive GC-rich stretches, termed GC1-4 (Figure 4D). This predicted structure consists of two RNA duplex regions (RDRs) formed by complementary pairings of GC1 to GC4 (RDRα) and GC2 to GC3 (RDRβ). The bases predicted to form RDRβ, but not the bases predicted to form RDRα are conserved in seed plants and seedless land plants (mosses and ferns) (Figure 4F). This suggests that the *ADC-box* is an ancient element of translational regulation that was present in non-vascular plants (Figure 4G), and that evolved further during speciation of seed plants. To study functional relevance of the GC-rich elements, we mutated GC1 (ΔGC1), GC2 (ΔGC1), or both GC-rich stretches (ΔGC1/2) within the long 5'UTR of native *SlADC1* transcripts and tested their translational activity. Individual mutations of either GC1 or GC2 moderately increased translational activity compared to the long 5'UTR of native *SlADC1* transcripts. The ΔGC1/2 double mutant had translational activity comparable to that of the short 5'UTR of Brg11-induced *SlADC1* transcripts (Figure 4E). No significant differences were observed for reporter mRNA levels, suggesting that reporter mRNAs do not differ in stability. Altogether, mutational analysis of the *ADC-box* demonstrates its inhibitory impact on translation and is consistent with the tentative RNA secondary structure.

### Example 4: Brg11 boosts host polyamine levels

ADCs catalyze conversion of arginine to agmatine, and this enzymatic activity provides a simple proxy for ADC expression. To determine ADC activity, the inventors established a mass spectrometry (MS) based assay in which conversion of ¹³C arginine to ¹³C agmatine was quantified in homogenized tissue of pathogen-infected tomato leaves. Delivery of Brg11 into tomato leaves via *R. solanacearum* or *X. euvesicatoria* resulted in 8- and 25-fold increase in ¹³C agmatine levels, respectively (Figures 5A and 5B). Importantly, Brg11 did not increase ¹³C agmatine levels in the tomato double mutant *Δ1*/*2-Brg11-EBE,* demonstrating that the integrity of the *Brg11-EBEs* upstream of *SlADC1*/*2* is crucial to *in planta* function of Brg11. The ADC inhibitor DL-α-difluoromethylarginine (DFMA) (Bitonti et al., 1987) reduced the observed increase in ¹³C agmatine by 4-fold, confirming that increase of ¹³C agmatine is the consequence of increased ADC activity. These results show that pathogen-mediated delivery of Brg11 into plant cells causes increased ADC activity.

In plants, the ADC product agmatine is converted to putrescine, a PA that serves either as a precursor for the longer PAs spermine and spermidine, or can be catabolized to γ-aminobutyric acid (GABA) (Liu et al., 2015; Tiburcio et al., 2014). To determine Brg11-induced changes in metabolite composition, tomato leaves were inoculated with *X. euvesicatoria* strains containing either Brg11* or Brg11ΔDBD* and the inventors profiled metabolites 24 hours post inoculation. MS-based analysis did not reveal significant Brg11-dependent changes for spermine, spermidine, or GABA levels. By contrast, Brg11 induced a five- and four-fold increase in agmatine and putrescine levels, respectively (Figures 5C-5G) which is consistent with the observed increase in ADC activity (Figures 5A and 5B). Since the observed cellular concentration of putrescine is about 100-fold higher than that of agmatine, it can be concluded that Brg11 is causing the most significant metabolic change in putrescine levels.

### Example 5: ADCs attenuate in planta growth of P. syringae

*R. solanacearum* strains containing or lacking Brg11 grew similarly in tomato leaves (Figure 6A) suggesting that elevated ADC activity is neither beneficial nor detrimental to in *planta* growth of *R. solanacearum.* The inventors studied *in planta* growth of the tomato leaf pathogens *X. euvesicatoria* and *Pseudomonas syringae.* While these two pathogens do not colonize roots and thus are unlikely direct niche competitors of *R. solanacearum,* they provide a conceptual test system to clarify if *ADC* gene activation can attenuate *in planta* growth of plant-associated microbes. First the inventors studied *in planta* growth of *X. euvesicatoria* strains containing either Brg11* or Brg11ΔDBD*, and found that both strains grew similarly in tomato leaves (Figure 6B). To see if *ADC* gene activation affects *in planta* growth of *P. syringae, P. syringae* was co-infiltrated with *X. euvesicatoria* strains delivering either Brg11* or Brg11ΔDBD*. It was found that *X. euvesicatoria* strains containing Brg11* but not strains containing Brg11ΔDBD* attenuated growth of co-cultivated *P. syringae* suggesting that activation of *SlADC1*/*2* has an inhibitory effect on *P. syringae* (Figure 6C). To obtain further evidence that *SlADC1*/*2* genes are involved in resistance against *P. syringae* CRISPR-Cas9 mutagenesis was used to induce heritable mutations in the CDS of *SlADC1*/*2.* Inoculation of tomato wild-type and the *Sladc1*/*2* double mutant revealed that the population size of *P. syringae* was almost ten-fold higher in the *Sladc1*/*2* double mutants, confirming that *SlADC1*/*2* genes are involved in plant resistance against *P. syringae* (Figure 6D). By contrast, population sizes of *R. solanacearum* and *X. euvesicatoria* were indistinguishable in *Sladc1*/*2* double mutants and tomato wild-type plants (Figures 6E and 6F). In summation, these findings demonstrate Brg11-mediated activation of *ADC* genes attenuates growth of microbes that are possibly detrimental to *R. solanacearum.*

### Example 6: Brg11 induces similar changes in root and leaf tissue

To clarify if Brg11-induced changes observed in tomato leaf cells occur also in tomato root cells, three different root inoculation assays were tested. In one of these assays, roots of mature (four-week old) tomato plants are inoculated by soil drenching (SD), an assay that mimics the natural infection process (Morel et al., 2018). The inventors also tested a recently established inoculation assay where roots of one-week old tomato seedlings are dipped into a bacterial suspension (SE) (Singh et al., 2018). In a variation of this assay, roots of tomato seedlings were inoculated via vacuum infiltration of bacterial suspensions (SEV) to promote delivery of bacteria into apoplastic cavities of the root. The inventors inoculated tomato roots by SD, SE and SEV assays, harvested tissue one and two days post inoculation and studied Brg11-induced changes at the transcript and at the metabolite level (Figures 7).

Qualitative analysis of root mRNA showed that Brg11 induces *ADC* transcripts with a short 5'UTR in all root samples irrespective of the chosen inoculation assay (Figures 7A [SE], 7B [SED] and 7C [SD]) which is consistent with the observations in leaf tissue (Figure 3A). Metabolic studies of root tissue inoculated by either SE or SEV assays showed a Brg11-dependent increase in ADC activity (Figures 7D). Root tissue inoculated by the SD assay also showed increased ADC activity. Brg11-dependent changes in agmatine and putrescine levels was studied and the it was found that both were increased in root tissue inoculated by the SEV assay (Figures 7E and 7F), but not in root tissue inoculated by SD or SE assays.

### Materials and Methods

Tomato cultivar Moneymaker was used as wild-type and for generation of mutant lines. *Sladc1*/*2* and Δ1/2-Brg11-EBE mutant tomato lines were generated by CRISPR-Cas9. Plants were grown in a growth chamber at 21 ± 1°C with a 16 h light/8 h dark photoperiod.

Wild-type eggplant cultivar Zebrina was used and grown in a growth chamber at 21 ± 1°C with a 16 h light/8 h dark photoperiod.

Wild-type *N. benthamiana* plants were grown in a growth chamber at 21 ± 2°C with a 16 h light/8 h dark photoperiod.

Wild-type *N. tabacum* plants were grown in a growth chamber at 21 ± 2°C with a 16 h light/8 h dark photoperiod.

The *R. solanacearum* wild-type strain GMI1000 (WT), brg11 mutant strain (Δbrg11) and hrcV mutant strain (ΔhrcV) were kind gifts from Stephane Genin and Nemo Peeters (LIPM, INRA, Toulouse, France). *R. solanacearum* were grown overnight in B medium (1% peptone, 0.1% tryptone, 0.1% yeast extract, 2.5% glucose) (for WT strain) or B medium supplemented with gentamycin (for Δbrg11 strain) or spectinomycin (for ΔhrcV strain) at 28°C. Bacterial cells were collected by centrifugation, and resuspended in sterile MiliQ water.

The *X. euvesicatoria* strains translocating Brg11* or Brg11ΔDBD* (also named as Hpx17) (Mukaihara et al., 2010) were generated as follows: the type III secretion signal (T3S signal) of AvrBs3 (N-terminal 152 amino acids) and the full-length coding sequence of Brg11 or Brg11ΔDBD were PCR amplified and cloned together into pENTR CACC-AAGG. pENTR carrying the above fragments were then digested with BamHI. The products at right size were gel purified and further ligated into BamHI digested pDSK602 vector in which contains the lac constitutive promoter in front of BamHI restriction site to drive the expression of the inserted genes. Cloned constructs were transformed into *X. euvesicatoria* 85-10 by electroporation. *X. euvesicatoria* strains delivering Brg11* or Brg11ΔDBD* were grown in NYG medium (0.5% peptone, 0.3% yeast extract, 2% glycerol) supplemented with spectinomycin and rifampicin at 28°C. Bacterial cells were collected by centrifugation and resuspended in sterile MiliQ water.

*P. syringae pv. tomato* DC3000 was kindly provided by Andrea Gust (ZMBP, University of Tübingen) and grown in NYG medium supplemented with rifampicin at 28°C. Bacterial cells were collected by centrifugation and resuspended in sterile MiliQ water.

### EBE prediction

EBE prediction was carried out using Talvez v.3.1 (Perez-Quintero et al. 2013) against putative promoter regions (1kb upstream of the translation start codon, both strands) of annotated tomato genes (SL3.0/ITAG3.10). The RVD-nucleotide matrix (Table S2) used for prediction was modified to allow pairings for Brg11 (e.g. "G" at the zero position) (de Lange et al., 2013). The score indicates the likelihood of the predicted Brg11-EBEs to be activated by Brg11.

### Promoter-uidA reporter assays

brg11 and avrBs3 were assembled into pGWB641 via Gateway Cloning. The pepper Bs3 promoter (343 bp upstream of ATG) and SlADC1/2 promoters (350 bp upstream of ATG) were first cloned into pUC57 and then transferred to pGWB3* via Golden Gate assembly (Binder et al., 2014). Candidate Brg11-EBEs and targeted mutations were integrated into the Bs3 promoter or SlADC1/2 promoters by PCR based mutagenesis. Primers used in this study were listed in Table S3.

All constructs were transformed into *A. tumefaciens* strain GV3101 by electroporation. A. tumefaciens were grown in YEB medium supplemented with spectinomycin and rifampicin (for pGWB641-containing strains), or kanamycin and rifampicin (for pGWB3*-containing strains) at 28°C for 24 h. Bacterial cells were collected by centrifugation, resuspended in inoculation medium (pH 5.6, 10 mM MgCl₂, 5 mM MES and 150 µM acetosyringone) and adjusted to an optical density at 600 nm (OD₆₀₀) of 0.8. Equal amount of A. tumefaciens strains delivering either the effector or the promoter-uidA fusions were mixed and infiltrated into 4-week-old N. benthamiana leaves with blunt-end syringe.

At 2 days post inoculation (dpi), leaf discs from infiltrated area were harvested and stained in GUS staining solution (pH= 7.0, 0.1 M sodium phosphate, 5 mM EDTA, 1 mM K₃[Fe(CN)₆], 1 mM K₄[Fe(CN)₆], 0.1% Triton X-100 and 0.05% X-Gluc) for 24 h, followed by washing in 70% ethanol for 2 days. Three representative stained leaf discs were scanned.

### Plant inoculation and quantitative real-time PCR

Four- to five-week-old tomato plants (Moneymaker or its corresponding mutants) were inoculated with either the R. solanacearum WT strain, Δbrg11 or ΔhrcV at OD₆₀₀ 0.1, and harvested at 18 hpi, or X. euvesicatoria strains expressing Brg11* or Brg11ΔDBD* at OD₆₀₀ 0.4 and harvested at 24 hpi. Five-week-old eggplant plants (Zebrina) were inoculated with the R. solanacearum WT strain, Δbrg11 and ΔhrcV at OD₆₀₀ 0.1, and harvested at 24 hpi. For A. tumefaciens mediated transient expression of Brg11 in N. benthamiana and N. tabacum, bacterial suspensions were adjusted to OD₆₀₀ 0.4 and infiltrated into four- to five-week-old plants. Infiltrated leaves were sampled at 2 dpi.

Total RNA was isolated from infiltrated tissue with the GeneMATRIX Universal RNA Purification Kit (EURx) according to the manufacturer's instruction. The quality of total RNA was checked by nanodrop. cDNA was synthesized from 500 ng of total RNA with the RevertAid First Strand cDNA Synthesis Kit (Thermo Scientific) according to the procedure provided by the company. Quantitative real-time PCR (qPCR) was performed in 8 µl reactions (BioRad, CFX384), each containing 1 pmol of forward and reverse primer, 2 µl of 1 to 20 diluted cDNA and 4 µl of MESA BLUE qPCR MasterMix Plus for SYBR® Assay (Eurogentec). The specificity of qPCR primers was checked by running a melting curve. TIP41 from tomato, 18S rRNA from eggplant, PP2A from N. benthamiana and EF 1α from N. tabacum were used as reference genes for internal normalization, respectively (Lacerda et al., 2015; Gantasala et al., 2013; Liu et al., 2012; Schmidt et al., 2010) . Relative expression was calculated with 2^{-ΔΔCT}.

### RNA sequencing and analysis

RNA quality was assessed with the Agilent 2100 Bioanalyzer system. Samples with RNA integrity number higher than 7.0 were selected for library generation. Poly(A) selected sequencing libraries were generated from 100 ng of total RNA using the NEBNext Ultra II Directional RNA Library Prep Kit for Illumina. All libraries were sequenced on the Illumina NextSeq 500 platform in single-end mode with read length 75 bp and at a depth of approx. 20 million clusters each. Library preparation and sequencing were performed by the same individual and a sequencing design aimed to minimize technical batch effects was chosen.

Quality of raw RNA-seq data in FASTQ files was assessed using ReadQC (ngs-bits version 2018_06) to identify potential sequencing cycles with low average quality and base distribution bias. Reads were preprocessed with skewer (version 0.2.2) and aligned using STAR (version 2.5.4a) allowing spliced read alignment to the tomato reference genome (version SL3.0, International Tomato Genome Sequencing Project). Alignment quality was analyzed using MappingQC (ngs-bits version 2018_06) and visually inspected with Broad Integrative Genome Viewer (version 2.4.11). Based on the genome annotation ITAG (version 3.20), read counts for all genes were obtained using subread (version 1.6.0).

For differential gene expression analysis, raw gene counts were filtered to only retain genes with at least 1 cpm (count per million) in at least half of the samples, leaving >17,000 genes for determining differential expression in each of the pair-wise comparisons between experimental groups. The analysis was performed with edgeR (version 3.22.3).

### CRISPR-CasQ mediated mutation in tomato

The one-vector CRISPR-Cas9 cloning system (p201N Cas9) (Jacobs et al., 2015) was used to mutate the Brg11-EBEs in wild-type tomato moneymaker. Since the Brg11-EBEs and their surrounding regions were conserved between SlADC1 and SlADC2 promoters, one guide RNA (gRNA) was designed to target the Brg11-EBEs in both promoters. The cloning of the gRNA into the T-DNA vector was done as previously described (Jacobs et al., 2015). The construct was introduced into A. tumefaciens strain by electroporation. A. tumefaciens containing both the gRNA and the Cas9 gene was transformed into tomato as previously described (Wittmann et al., 2016). For screening of mutant lines, total genomic DNA was extracted from individual lines and the regions containing the target sites were PCR amplified and sequenced. The mutant line (#20) that had homozygous mutations in Brg11-EBEs of both SlADC1/2 promoters was kept for producing seeds. In the next generation, Cas9 transgene free plants were screened by PCR amplification of the Cas9 coding sequence. The inoculation assay was performed on T3 progeny. The generation of SlADC1 and SlADC2 CDS single mutants (Sladc1 and Sladc2) were the same as for the Brg11-EBEs mutant except that two gRNAs were designed for each target gene. The SlADC1/2 double mutants (Sladc1/2) were obtained by crossing Sladc1 with Sladc2. F3 generation were used for inoculation analysis.

### 5' Rapid amplification of cDNA ends

5'RACE was carried out with the SMARTerTM RACE cDNA Amplification Kit (Clontech). Briefly, high quality RNA samples were synthesized into first-strand cDNA with SMARTScribeTM Reverse Transcriptase. PCR were then performed with PrimeSTAR GXL DNA Polymerase (Clontech) using diluted cDNA as template. To improve the specificity of the PCR products, nested PCR were performed in all cases. PCR products from 5'RACE PCR were gel purified and cloned into the commercial cloning vector CloneJET (Thermo Fisher Scientific). Several colonies from each cloning were sequenced and analyzed.

### RNA secondary structure prediction for the ADC-box

Based on the highly conserved Brg11-EBE sequence a multiple-sequence alignment was created using the plant subset of the RefSeq nucleotide database (O'Leary et al., 2016). A Picea abies sequence was also extracted from GenBank. To find additional homologs of this region, we performed repeated searches using the Infernal software, version 1.1 (Nawrocki et al., 2013). By using the CMfinder software (Yao et al., 2006), potential conserved secondary structures were analyzed. To evaluate genes associated with the conserved element, we used genome annotations available in RefSeq and domain predictions from the conserved domain database (Marchler-Bauer et al., 2017) version 2.25. Predicted elements adjacent to annotated pseudogenes were not investigated further. Notably, no base pairs in the proposed structure are convincingly supported by covariation (Michel et al., 1990), i.e. mutations that modify both nucleotides in a base pair, yet preserve Watson-Crick pairing. Additionally, some ADC-box sequences contain base pairs that are not usually energetically favorable in Watson-Crick pairs. For these reasons, the proposed structure must be regarded as tentative.

### In vitro translation

Different 5'UTRs were fused to the GFP reporter gene and driven by the T7 promoter. Capped mRNA was transcribed in vitro in the presence of the cap analog m27,3'-OG[5']ppp[5']G (Jena Bioscience, Jena, Germany) using the HiScribe™ T7 High Yield RNA Synthesis Kit (New England Biolabs, Ipswich, MA, USA). Either PCR products amplified by Phusion polymerase (New England Biolabs) or plasmid DNA linearized with EcoRI were used as template for RNA synthesis. The RNA was purified using the DyeEx 2.0 Spin Kit (Qiagen, Hilden, Germany).

In vitro translation was carried out in a 50 µl scale containing 50% (v/v) tobacco BY-2 cell-free lysates, 16% (v/v) TR buffer (30 mM HEPES-KOH, pH 7.6, 40 mM potassium glutamate, 0.5 mM magnesium glutamate, 2 mM DTT), 0.75 mM ATP, 0.1 mM GTP, 25 mM creatine phosphate, 50 µM of each amino acid, 0.1 mg/ml creatine phosphokinase (Roche Diagnostics) and 8.5 pmol purified mRNA. The magnesium concentration was adjusted to 1.5 mM with magnesium glutamate and the potassium concentration was adjusted to 38 mM with potassium glutamate. Any existing magnesium and potassium in tobacco BY-2 cell-free lysates were ignored. The batch translation reactions were carried out in a LT-X (Lab-Therm) shaker (Kuhner, Birsfelden, Switzerland) at 25°C and 500 rpm. The GFP fluorescence signal from each reaction were quantified using a Synergy HT Multi-Mode Microplate Reader (Biotek, Bad Friedrichshall, Germany) with 485/20 nm excitation and 528/20 nm emission.

### In vivo translational assay

A GFP/DsRed dual fluorescence reporter system was used for different 5'UTRs-driven reporter gene translational studies. The constructs were assembled by Golden Gate Cloning (Binder et al., 2014). Firstly, 35S promoter, 5'UTRs, GFP, DsRed, 35S terminator were cloned into LI vector. Taking advantage of Golden Gate Cloning, GATG, in which G is the last base of the 5'UTRs and ATG is the first three bases of GFP, was defined as overhang between the 5'UTRs and GFP so that those two fragments were seamlessly connected without introducing extra bases. Secondly, individual functional units expressing GFP or DsRed were assembled as LII vector. GFP was designed as the translational reporter and DsRed was used as the internal control. Lastly, the assembled LII vectors were combined into a LIII binary vector for expression of both reporters within one T-DNA.

The plasmids were transformed into A. tumefaciens by electroporation. A. tumefaciens mediated reporter gene transient expression in N. benthamiana was performed as described above. Leaf tissue were harvested at 36 hpi for fluorescence scanning and quantification. For GFP and DsRed protein quantification, 6 leaf discs (0.25 cm²) were ground in liquid nitrogen and total protein was extracted in 300 µl of isolation buffer (50 mM Tris-HCl, pH=7.5, 150 mM NaCl, 0.1% Tween 20, 0.1% β-mercaptoethanol) as previously described (Stauffer et al., 2010). GFP and DsRed intensity from protein extracts were measured using a fluorometer (Berthold Plate Reader).

### Bacterial growth assay

For growth curve of R. solanacearum, X. euvesicatoria and P. syringae, bacteria suspensions were adjusted to 10⁵ CFU/ml with sterile water and infiltrated into 4- to 5-week-old tomato leaves. Inoculated plants were incubated at 27°C with a 12-h light cycle (R. solanacearum) or 21 ± 1°C with a 16h/8h day/night cycle (X. euvesicatoria and P. syringae). Leaf discs were harvested at 2, 3 or 4 dpi and ground in MiliQ water in a Retsch MM200 (25 f) for 1 min. Series of dilutions were plated out on agar plate and incubated at 28°C for 36 to 48 h.

For competitive growth of P. syringae and X. euvesicatoria, P. syringae were adjusted to 10⁵ CFU/ml and X. euvesicatoria were adjusted to 10⁸ CFU/ml. Equal volume of P. syringae and X. euvesicatoria were mixed and infiltrated into 4- to 5-week-old tomato leaves. Inoculated plants were incubated at 21 ± 1°C with a 16h/8h day/night cycle. Bacteria growth were measured as described above.

### ADC activity assay and polyamine analysis

Bacteria infected tissue were homogenized in ADC activity isolation buffer (5 mM Tris, pH 7.5, 1 mM ascorbate, 50 µM pyridoxal 5 phosphate (Sigma Aldrich), 5% polyvinylpyrolidine) (3 µl buffer/mg tissue). After centrifugation at 17,000 g for 20 min at 4°C, supernatants were carefully transferred to a new tube and kept on ice. ADC activity assays were set up as follows: 90 µl of protein extract was combined with either 5 µl of Milli-Q water or 5 µl of 1 mM DL-α-(Difluoromethyl) arginine (DFMA; Santa Cruz Biotechnology, Germany). The reaction was started by adding 5 µl of 1 mM U-13C6 L-arginine (Euroisotop, Germany). After incubation at 37°C for 1 h - gently mixing - the enzymatic reaction was stopped with 5 µl of heptafluorobutyric acid (HFBA, pKs=0.4; Sigma Aldrich) and centrifuged at 17,000 g for 20 min at 10°C. The supernatant was diluted either 1 to 5 or 1 to 10 and subjected to metabolite analysis.

The LC-MRM-MS profiling was performed as reported previously (Sanchez-Lopez et al., 2009) using an Ekspert Micro-LC 200 and a QTRAP4000 (ABSciex, USA). Chromatographic separation was achieved on a HaloFused C18 column (150 x 0.5 mm; 2.7 µm; AB Sciex) applying the following binary gradient at a flow rate of 11 µl/min and 35°C:0 - 0.5 min isocratic 90% A; 0.5 - 4 min, linear from 90% A to 1% A; 4 - 4.8 min, isocratic 1% A (water, 0.1% aq. formic acid, 0.05% heptafluorobutyric acid); 4.8-5 min, linear from 1% A to 90% A; 5-5.5 min, linear to 99% A; 5.5-5.8 min, linear to 90% A; 5.8- 6 min, isocratic 10% B (acetonitrile, 0.1% aq. formic acid, 0.05% heptafluorobutyric acid). The injection volume was 2 µl. Analytes were ionized using a TurboV ion source equipped with an Assy 65 µm ESI electrode in positive ion mode. The following instrument settings were applied: nebulizer and heater gas, nitrogen, 25 and 10 psi; curtain gas, nitrogen, 20 psi; collision gas, nitrogen, medium; source temperature, 200°C; ionspray voltage, 5000 V; entrance potential, 10 V; collision cell exit potential, 5V; scan time 25msec. The transitions monitored for each analyte were listed in Table S4. The MRM data acquired were analyzed using the vendors MultiQuant software.

### Quantification and Statistical Analysis

Statistical analysis was performed in R (R Version 3.3.3). Where applicable, the Student's t-test and one-way ANOVA followed by Tukey's HSD test were used to compare significance levels between different groups. All statistical details can be found in the figure legends. The data are presented as mean ± SD. n represents number of plants.

### REFERENCES

The references are:
Albert, I., Bohm, H., Albert, M., Feiler, C.E., Imkampe, J., Wallmeroth, N., Brancato, C., Raaymakers, T.M., Oome, S., Zhang, H.Q., et al. (2015). An RLP23-SOBIR1-BAK1 complex mediates NLP-triggered immunity. Nature Plants. 1, 15140.
Alcazar, R., and Tiburcio, A.F. (2014). Plant polyamines in stress and development: an emerging area of research in plant sciences. Front. Plant Sci. 5, 319
Barrett, L.W., Fletcher, S., and Wilton, S.D. (2012). Regulation of eukaryotic gene expression by the untranslated gene regions and other non-coding elements. Cell. Mol. Life Sci. 69, 3613-3634.
Binder, A., Lambert, J., Morbitzer, R., Popp, C., Ott, T., Lahaye, T., and Parniske, M. (2014). A modular plasmid assembly kit for multigene expression, gene silencing and silencing rescue in plants. PLoS One. 9, e88218.
Bitonti, A.J., Casara, P.J., McCann, P.P., and Bey, P. (1987). Catalytic irreversible inhibition of bacterial and plant arginine decarboxylase activities by novel substrate and product analogs. Biochem. J. 242, 69-74
Boch, J., and Bonas, U. (2010). Xanthomonas AvrBs3 family-type III effectors: discovery and function. Annu. Rev. Phytopathol. 418, 419-436.
Boch, J., Bonas, U., and Lahaye, T. (2014). TAL effectors - pathogen strategies and plant resistance engineering. New Phytol. 204, 823-832.
Buntru, M., Vogel, S., Spiegel, H., and Schillberg, S. (2014). Tobacco BY-2 cell-free lysate: an alternative and highly-productive plant-based in vitro translation system. BMC Biotechnol. 14, 37
Buntru, M., Vogel, S., Stoff, K., Spiegel, H., and Schillberg, S. (2015). A versatile coupled cell-free transcription-translation system based on tobacco BY-2 cell lysates. Biotechnol. Bioeng. 112, 867-878.
Büttner, D. (2016). Behind the lines-actions of bacterial type III effector proteins in plant cells. FEMS Microbiol. Rev. 40, 894-937.
Chen, D.D., Shao, Q.S., Yin, L.H., Younis, A., and Zheng, B.S. (2019). Polyamine function in plants: metabolism, regulation on development, and roles in abiotic stress responses. Front. Plant Sci. 9, 1945
de Lange, O., Schreiber, T., Schandry, N., Radeck, J., Braun, K.H., Koszinowski, J., Heuer, H., Strauß, A., and Lahaye, T. (2013). Breaking the DNA binding code of Ralstonia solanacearum TAL effectors provides new possibilities to generate plant resistance genes against bacterial wilt disease. New Phytol. 199, 773-786.
Denancé, N., Szurek, B., Doyle, E.L., Lauber, E., Fontaine-Bodin, L., Carrère, S., Guy, E., Hajri, A., Cerutti, A., Boureau, T., et al. (2018). Two ancestral genes shaped the Xanthomonas campestris TAL effector gene repertoire. New Phytol. 219, 391-407.
El Kasmi, F., Horvath, D., and Lahaye, T. (2018). Microbial effectors and the role of water and sugar in the infection battle ground. Curr. Opin. Plant Biol. 44, 98-107.
Galán, J.E., and Waksman, G. (2018). Protein-injection machines in bacteria. Cell. 172, 1306-1318.
Gantasala, N.P., Papolu, P.K., Thakur, P.K., Kamaraju, D., Sreevathsa, R., and Rao, U. (2013). Selection and validation of reference genes for quantitative gene expression studies by real-time PCR in eggplant (Solanum melongena L). BMC research notes. 6, 312.
Garcia-Bayona, L., and Comstock, L.E. (2018). Bacterial antagonism in host-associated microbial communities. Science. 361, 361.
Granato, E.T., Meiller-Legrand, T.A., and Foster, K.R. (2019). The evolution and ecology of bacterial warfare. Curr. Biol. 29, R521-R537.
Guidot, A., Jiang, W., Ferdy, J.B., Thébaud, C., Barberis, P., Gouzy, J., and Genin, S. (2014). Multihost experimental evolution of the pathogen Ralstonia solanacearum unveils genes involved in adaptation to plants. Mol. Biol. Evol. 31, 2913-2928.
Hanfrey, C., Sommer, S., Mayer, M.J., Burtin, D., and Michael, A.J. (2001). Arabidopsis polyamine biosynthesis: absence of ornithine decarboxylase and the mechanism of arginine decarboxylase activity. Plant J. 27, 551-560.
Henry, E., Toruno, T.Y., Jauneau, A., Deslandes, L., and Coaker, G. (2017). Direct and indirect visualization of bacterial effector delivery into diverse plant cell types during infection. Plant Cell. 29, 1555-1570.
Jacobs, T.B., LaFayette, P.R., Schmitz, R.J., and Parrott, W.A. (2015). Targeted genome modifications in soybean with CRISPR/Cas9. BMC Biotechnol. 15, 16.
Jimenez-Bremont, J.F., Marina, M., Guerrero-Gonzalez, M.D., Rossi, F.R., Sanchez-Rangel, D., Rodriguez-Kessler, M., Ruiz, O., and Garriz, A. (2014). Physiological and molecular implications of plant polyamine metabolism during biotic interactions. Front. Plant Sci. 5, 95.
Jwa, N.S., and Hwang, B.K. (2017). Convergent evolution of pathogen effectors toward reactive oxygen species signaling networks in plants. Front. Plant Sci. 8, 1687.
Kay, S., Hahn, S., Marois, E., Hause, G., and Bonas, U. (2007). A bacterial effector acts as a plant transcription factor and induces a cell size regulator. Science. 318, 648-651.
Kim, N.H., Kim, B.S., and Hwang, B.K. (2013). Pepper arginine decarboxylase is required for polyamine and γ-aminobutyric acid signaling in cell death and defense response. Plant Physiol. 162, 2067-2083.
Kim, S.H., Kim, S.H., Yoo, S.J., Min, K.H., Nam, S.H., Cho, B.H., and Yang, K.Y. (2013). Putrescine regulating by stress-responsive MAPK cascade contributes to bacterial pathogen defense in Arabidopsis. Biochem. Biophys. Res. Commun. 437, 502-508.
Kwak, M.-J., Kong, H.G., Choi, K., Kwon, S.-K., Song, J.Y., Lee, J., Lee, P.A., Choi, S.Y., Seo, M., Lee, H.J., et al. (2018). Rhizosphere microbiome structure alters to enable wilt resistance in tomato. Nat. Biotechnol. 36, 1100.
Lacerda, A.L.M., Fonseca, L.N., Blawid, R., Boiteux, L.S., Ribeiro, S.G., and Brasileiro, A.C.M. (2015). Reference gene selection for qPCR analysis in tomato-bipartite begomovirus interaction and validation in additional tomato-virus pathosystems. PLoS One. 10, e0136820
Liu, C., Atanasov, K.E., Tiburcio, A.F., and Alcázar, R. (2019). The polyamine putrescine contributes to H2O2 and RbohD/F-dependent positive feedback loop in Arabidopsis PAMP-triggered immunity. Front. Plant Sci. 10, 10.3389.
Liu, D.S., Shi, L.D., Han, C.G., Yu, J.L., Li, D.W., and Zhang, Y.L. (2012). Validation of reference genes for gene expression studies in virus-infected Nicotiana benthamiana using quantitative real-time PCR. PLoS One. 7, e46451
Liu, J.H., Wang, W., Wu, H., Gong, X.Q., and Moriguchi, T. (2015). Polyamines function in stress tolerance: from synthesis to regulation. Front. Plant Sci. 6, 10.3389.
Lou, Y.R., Bor, M., Yan, J., Preuss, A.S., and Jander, G. (2016). Arabidopsis NATA1 acetylates putrescine and decreases defense-related hydrogen peroxide accumulation. Plant Physiol. 171, 1443-1455.
Lowe-Power, T.M., Hendrich, C.G., von Roepenack-Lahaye, E., Li, B., Wu, D., Mitra, R., Dalsing, B.L., Ricca, P., Naidoo, J., Cook, D., et al. (2018). Metabolomics of tomato xylem sap during bacterial wilt reveals Ralstonia solanacearum produces abundant putrescine, a metabolite that accelerates wilt disease. Environ Microbiol. 20, 1330-1349.
Ma, L.S., Hachani, A., Lin, J.S., Filloux, A., and Lai, E.M. (2014). Agrobacterium tumefaciens deploys a superfamily of type VI secretion DNase effectors as weapons for interbacterial competition in planta. Cell Host Microbe. 16, 94-104.
Mach, J. (2017). Tracking the bacterial type III secretion system: visualization of effector delivery using split fluorescent proteins. Plant Cell. 29, 1547-1548.
Macho, A.P., Guidot, A., Barberis, P., Beuzon, C.R., and Genin, S. (2010). A competitive index assay identifies several Ralstonia solanacearum type III effector mutant strains with reduced fitness in host plants. Mol. Plant Microbe Interact. 23, 1197-1205.
Marchler-Bauer, A., Bo, Y., Han, L., He, J., Lanczycki, C.J., Lu, S., Chitsaz, F., Derbyshire, M.K., Geer, R.C., Gonzales, N.R., et al. (2017). CDD/SPARCLE: functional classification of proteins via subfamily domain architectures. Nucleic Acids Res. 45, D200-D203.
Michel, F., and Westhof, E. (1990). Modelling of the three-dimensional architecture of group I catalytic introns based on comparative sequence analysis. J. Mol. Biol. 216, 585-610.
Mittler, R. (2017). ROS Are Good. Trends Plant Sci. 22, 11-19.
Morel, A., Peeters, N., Vailleau, F., Barberis, P., Jiang, G.F., Berthome, R., and Guidot, A. (2018). Plant pathogenicity phenotyping of Ralstonia solanacearum strains. Host-Pathogen Interactions: Methods and Protocols. 1734, 223-239.
Mukaihara, T., Tamura, N., and Iwabuchi, M. (2010). Genome-wide identification of a large repertoire of Ralstonia solanacearum type III effector proteins by a new functional screen. Mol Plant Microbe Interact. 23, 251-262.
Nawrocki, E.P., and Eddy, S.R. (2013). Infernal 1.1: 100-fold faster RNA homology searches. Bioinformatics. 29, 2933-2935.
O'Leary, N.A., Wright, M.W., Brister, J.R., Ciufo, S., Haddad, D., McVeigh, R., Rajput, B., Robbertse, B., Smith-White, B., Ako-Adjei, D., et al. (2016). Reference sequence (RefSeq) database at NCBI: current status, taxonomic expansion, and functional annotation. Nucleic Acids Res. 44, D733-745.
O'Neill, E.M., Mucyn, T.S., Patteson, J.B., Finkel, O.M., Chung, E.H., Baccile, J.A., Massolo, E., Schroeder, F.C., Dangl, J.L., and Li, B. (2018). Phevamine A, a small molecule that suppresses plant immune responses. Proc. Natl. Acad. Sci. USA. 115, E9514-E9522.
Park, E., Lee, H.Y., Woo, J., Choi, D., and Dinesh-Kumar, S.P. (2017). Spatiotemporal monitoring of Pseudomonas syringae effectors via type III secretion using split fluorescent protein fragments. Plant Cell. 29, 1571-1584.
Perez-Quintero, A.L., Rodriguez, R.L., Dereeper, A., Lopez, C., Koebnik, R., Szurek, B., and Cunnac, S. (2013). An improved method for TAL effectors DNA-binding sites prediction reveals functional convergence in TAL repertoires of Xanthomonas oryzae strains. PLoS One. 8, e68464.
Prior, P., and Steva, H. (1990). Characteristics of strains of Pseudomonas solanacearum from the French West-Indies. Plant Dis. 74, 13-17.
Qi, J., Wang, J., Gong, Z., and Zhou, J.M. (2017). Apoplastic ROS signaling in plant immunity. Curr. Opin. Plant Biol. 38, 92-100.
Römer, P., Strauss, T., Hahn, S., Scholze, H., Morbitzer, R., Grau, J., Bonas, U., and Lahaye, T. (2009). Recognition of AvrBs3-like proteins is mediated by specific binding to promoters of matching pepper Bs3 alleles. Plant Physiol. 150, 1697-1712.
Rovenich, H., Boshoven, J.C., and Thomma, B.P.H.J. (2014). Filamentous pathogen effector functions: of pathogens, hosts and microbiomes. Curr. Opin. Plant Biol. 20, 96-103.
Rüter, C., Lubos, M.L., Norkowski, S., and Schmidt, M.A. (2018). All in - multiple parallel strategies for intracellular delivery by bacterial pathogens. Int. J. Med. Microbiol. 308, 872-881.
Salanoubat, M., Genin, S., Artiguenave, F., Gouzy, J., Mangenot, S., Arlat, M., Billault, A., Brottier, P., Camus, J.C., Cattolico, L., et al. (2002). Genome sequence of the plant pathogen Ralstonia solanacearum. Nature. 415, 497-502.
Sanchez-Lopez, J., Camanes, G., Flors, V., Vicent, C., Pastor, V., Vicedo, B., Cerezo, M., and Garcia-Agustin, P. (2009). Underivatized polyamine analysis in plant samples by ion pair LC coupled with electrospray tandem mass spectrometry. Plant Physiol. Bioch. 47, 592-598.
Schandry, N., de Lange, O., Prior, P., and Lahaye, T. (2016). TALE-like effectors are an ancestral feature of the Ralstonia solanacearum species complex and converge in DNA targeting specificity. Front. Plant Sci. 7, 1225.
Schmidt, G.W., and Delaney, S.K. (2010). Stable internal reference genes for normalization of real-time RT-PCR in tobacco (Nicotiana tabacum) during development and abiotic stress. Mol. Genet. Genomics. 283, 233-241.
Singh, N., Phukan, T., Sharma, P.L., Kabyashree, K., Barman, A., Kumar, R., Sonti, R., Genin, S., and Ray, S.K. (2018). An innovative root inoculation method to study Ralstonia solanacearum pathogenicity in tomato seedlings. Phytopathology. 108, 436-442.
Souza, D.P., Oka, G.U., Alvarez-Martinez, C.E., Bisson-Filho, A.W., Dunger, G., Hobeika, L., Cavalcante, N.S., Alegria, M.C., Barbosa, L.R., Salinas, R.K., et al. (2015). Bacterial killing via a type IV secretion system. Nat. Commun. 6, 6453.
Stauffer, E., Westermann, A., Wagner, G., and Wachter, A. (2010). Polypyrimidine tract-binding protein homologues from Arabidopsis underlie regulatory circuits based on alternative splicing and downstream control. Plant J. 64, 243-255.
Tiburcio, A.F., Altabella, T., Bitrian, M., and Alcazar, R. (2014). The roles of polyamines during the lifespan of plants: from development to stress. Planta. 240, 118.
Urano, K., Hobo, T., and Shinozaki, K. (2005). Arabidopsis ADC genes involved in polyamine biosynthesis are essential for seed development. FEBS Lett. 579, 1557-1564.
von Arnim, A.G., Jia, Q.D., and Vaughn, J.N. (2014). Regulation of plant translation by upstream open reading frames. Plant Science. 214, 1-12.
Wachter, A. (2014). Gene regulation by structured mRNA elements. Trends Genet. 30, 172-181.
Wittmann, J., Brancato, C., Berendzen, K.W., and Dreiseikelmann, B. (2016). Development of a tomato plant resistant to Clavibacter michiganensis using the endolysin gene of bacteriophage CMP1 as a transgene. Plant Pathology. 65, 496-502.
Yao, Z.Z., Weinberg, Z., and Ruzzo, W.L. (2006). CMfinder - a covariance model based RNA motif finding algorithm. Bioinformatics. 22, 445-452.

## Claims

1. **A method for modulating the translation of a target messenger RNA (mRNA),** the method comprising mutating at least one nucleotide in a genetic elementcontained in the 5' untranslated region encoding said mRNA, wherein said genetic element comprises in successive order GC rich regions GC2 which is encoded by the sequence CCG(A/G)GGC and GC3 which is encoded by the sequence GCCTCGG.

2. The method according to claim 1, wherein said genetic element comprises in successive order four GC rich regions GC1, GC2, GC3 and GC4, and wherein GC1 is encoded by the sequence GGGGG(T/A); and GC4 is encoded by the sequence (G/-)CCCC(C/T).

3. The method according to claim 1 or 2, wherein the at least one nucleotide is located within any one of GC1 to GC4, and/or within a sequence between any two of GC1 to GC4.

4. The method according to any one of claims 1 to 3, wherein the genetic element is within the 5' untranslated region of an *argenine decarboxylase* (*ADC*) gene, and wherein said mRNA is an mRNA of an *ADC.*

5. The method according to any one of claims 1 to 4, wherein GC1 and GC2 are separated by two nucleotides, preferably AG, such that the genetic element comprises a sequence encoded by: GGGGG(T/A)AGCCG(A/G)GGC (or GGGGG(U/A)AGCCG(A/G)GGC as mRNA), and preferably is GGGGGTAGCCGGGGC.

6. **A method for modulating the expression of a protein coding gene,** the method comprising introducing into the 5'untranslated region of the transcribed region of the protein coding gene an ADC-box, or a fragment thereof, to obtain an ADC-box modified protein coding gene, wherein said ADC-box, or a fragment thereof, comprises in successive (5' to 3') order GC rich regions GC2 which comprises the sequence CCG-A/G-GGC and GC3 which comprises the sequence GCCTCGG; and preferably wherein the ADC box is introduced between the transcriptional start and translational start of said 5' untranslated region.

7. The method according to claim 6, wherein modulating the expression of a protein coding gene is a decrease in the level of protein translation of the mRNA transcribed from said protein coding gene, and/or a decrease in the level of protein translated from the mRNA transcribed from said protein coding gene, and/or is an increase of mRNA decay of the mRNA transcribed from said protein coding gene.

8. **An isolated nucleic acid comprising an ADC-box, or a fragment thereof,** wherein said nucleic acid comprises an ADC-box sequence, or a fragment thereof, comprising in successive (5' to 3') order GC rich regions GC2 which comprises the sequence CCG(A/G)GGC and GC3 which comprises the sequence GCCTCGG.

9. The isolated nucleic acid according to claim 8, wherein said genetic element comprises in successive order four GC rich regions GC1, GC2, GC3 and GC4, and wherein GC1 is encoded by the sequence GGGGG(T/A); and GC4 is encoded by the sequence (G/)CCCC(C/T).

10. **A system for the modulation of protein expression,** the system comprising (i) a translation attenuation component which is an isolated nucleic acid according to any one of claims 8 or 9, and (ii) a transcriptional start modification component which is nucleic acid for the expression of a TALE protein, or a TALE protein; and preferably wherein the TALE protein is Brg11.

11. **A method for increasing ADC activity in a cell,** the method comprising introducing into the cell Brg11, or a variant thereof, or performing a method according to any one of claims 1 to 7.

12. **A method for producing a plant with increased ADC activity,** the method comprising modulating the expression of an ADC mRNA by a method according to any one of claims 1 to 7.

13. **A transgenic plant with modulated ADC gene expression,** the transgenic plant comprising an ADC gene comprising a mutated ADC-box as recited in any one of claims 1 to 7.

14. **A method for modulating an immune response of a plant or plant cell against pathogens which are sensitive to a polyamine mediated plant immune response,** the method comprising the steps of a method according to any one of claims 11 or 12.

15. **A method for modulating gene expression,** the method comprising
(i) Providing a target gene having in its 5' untranslated region, preferably between the transcriptional start and translational start, a translation regulatory element the presence of which in the transcribed mRNA results in a modulated translation of said mRNA,
(ii) Contacting the target gene with a TALE protein and thereby induce a transcriptional start of the target gene in 3' direction of the regulatory element within the 5' untranslated region.


SEQUENCE LISTING

<110>  Eberhart Karls Universität Tübingen

<120>  REGULATORY ELEMENT OF AN ARGININE DECARBOXYLASE GENE AND METHODS
       AND USES THEREOF

<130>  U30939EP

<160>  27

<170>  PatentIn version 3.5

<210>  1
<211>  1245
<212>  PRT
<213>  Ralstonia solanacearum

<400>  1

Met Arg Ile Gly Lys Ser Ser Gly Trp Leu Asn Glu Ser Val Ser Leu
1               5                   10                  15

Glu Tyr Glu His Val Ser Pro Pro Thr Arg Pro Arg Asp Thr Arg Arg
            20                  25                  30

Arg Pro Arg Ala Ala Gly Asp Gly Gly Leu Ala His Leu His Arg Arg
        35                  40                  45

Leu Ala Val Gly Tyr Ala Glu Asp Thr Pro Arg Thr Glu Ala Arg Ser
    50                  55                  60

Pro Ala Pro Arg Arg Pro Leu Pro Val Ala Pro Ala Ser Ala Pro Pro
65                  70                  75                  80

Ala Pro Ser Leu Val Pro Glu Pro Pro Met Pro Val Ser Leu Pro Ala
                85                  90                  95

Val Ser Ser Pro Arg Phe Ser Ala Gly Ser Ser Ala Ala Ile Thr Asp
            100                 105                 110

Pro Phe Pro Ser Leu Pro Pro Thr Pro Val Leu Tyr Ala Met Ala Arg
        115                 120                 125

Glu Leu Glu Ala Leu Ser Asp Ala Thr Trp Gln Pro Ala Val Pro Leu
    130                 135                 140

Pro Ala Glu Pro Pro Thr Asp Ala Arg Arg Gly Asn Thr Val Phe Asp
145                 150                 155                 160

Glu Ala Ser Ala Ser Ser Pro Val Ile Ala Ser Ala Cys Pro Gln Ala
                165                 170                 175

Phe Ala Ser Pro Pro Arg Ala Pro Arg Ser Ala Arg Ala Arg Arg Ala
        180                 185                 190

Arg Thr Gly Gly Asp Ala Trp Pro Ala Pro Thr Phe Leu Ser Arg Pro
    195                 200                 205

Ser Ser Ser Arg Ile Gly Arg Asp Val Phe Gly Lys Leu Val Ala Leu
    210                 215                 220

Gly Tyr Ser Arg Glu Gln Ile Arg Lys Leu Lys Gln Glu Ser Leu Ser
225                 230                 235                 240

Glu Ile Ala Lys Tyr His Thr Thr Leu Thr Gly Gln Gly Phe Thr His
                245                 250                 255

Ala Asp Ile Cys Arg Ile Ser Arg Arg Arg Gln Ser Leu Arg Val Val
            260                 265                 270

Ala Arg Asn Tyr Pro Glu Leu Ala Ala Ala Leu Pro Glu Leu Thr Arg
        275                 280                 285

Ala His Ile Val Asp Ile Ala Arg Gln Arg Ser Gly Asp Leu Ala Leu
    290                 295                 300

Gln Ala Leu Leu Pro Val Ala Thr Ala Leu Thr Ala Ala Pro Leu Arg
305                 310                 315                 320

Leu Ser Ala Ser Gln Ile Ala Thr Val Ala Gln Tyr Gly Glu Arg Pro
                325                 330                 335

Ala Ile Gln Ala Leu Tyr Arg Leu Arg Arg Lys Leu Thr Arg Ala Pro
            340                 345                 350

Leu His Leu Thr Pro Gln Gln Val Val Ala Ile Ala Ser Asn Thr Gly
        355                 360                 365

Gly Lys Arg Ala Leu Glu Ala Val Cys Val Gln Leu Pro Val Leu Arg
    370                 375                 380

Ala Ala Pro Tyr Arg Leu Ser Thr Glu Gln Val Val Ala Ile Ala Ser
385                 390                 395                 400

Asn Lys Gly Gly Lys Gln Ala Leu Glu Ala Val Lys Ala His Leu Leu
                405                 410                 415

Asp Leu Leu Gly Ala Pro Tyr Val Leu Asp Thr Glu Gln Val Val Ala
            420                 425                 430

Ile Ala Ser His Asn Gly Gly Lys Gln Ala Leu Glu Ala Val Lys Ala
        435                 440                 445

Asp Leu Leu Asp Leu Arg Gly Ala Pro Tyr Ala Leu Ser Thr Glu Gln
    450                 455                 460

Val Val Ala Ile Ala Ser His Asn Gly Gly Lys Gln Ala Leu Glu Ala
465                 470                 475                 480

Val Lys Ala Asp Leu Leu Glu Leu Arg Gly Ala Pro Tyr Ala Leu Ser
                485                 490                 495

Thr Glu Gln Val Val Ala Ile Ala Ser His Asn Gly Gly Lys Gln Ala
            500                 505                 510

Leu Glu Ala Val Lys Ala His Leu Leu Asp Leu Arg Gly Val Pro Tyr
        515                 520                 525

Ala Leu Ser Thr Glu Gln Val Val Ala Ile Ala Ser His Asn Gly Gly
    530                 535                 540

Lys Gln Ala Leu Glu Ala Val Lys Ala Gln Leu Leu Asp Leu Arg Gly
545                 550                 555                 560

Ala Pro Tyr Ala Leu Ser Thr Ala Gln Val Val Ala Ile Ala Ser Asn
                565                 570                 575

Gly Gly Gly Lys Gln Ala Leu Glu Gly Ile Gly Glu Gln Leu Leu Lys
            580                 585                 590

Leu Arg Thr Ala Pro Tyr Gly Leu Ser Thr Glu Gln Val Val Ala Ile
        595                 600                 605

Ala Ser His Asp Gly Gly Lys Gln Ala Leu Glu Ala Val Gly Ala Gln
    610                 615                 620

Leu Val Ala Leu Arg Ala Ala Pro Tyr Ala Leu Ser Thr Glu Gln Val
625                 630                 635                 640

Val Ala Ile Ala Ser Asn Lys Gly Gly Lys Gln Ala Leu Glu Ala Val
                645                 650                 655

Lys Ala Gln Leu Leu Glu Leu Arg Gly Ala Pro Tyr Ala Leu Ser Thr
            660                 665                 670

Ala Gln Val Val Ala Ile Ala Ser His Asp Gly Gly Asn Gln Ala Leu

675                 680                 685

Glu Ala Val Gly Thr Gln Leu Val Ala Leu Arg Ala Ala Pro Tyr Ala
    690                 695                 700

Leu Ser Thr Glu Gln Val Val Ala Ile Ala Ser His Asp Gly Gly Lys
705                 710                 715                 720

Gln Ala Leu Glu Ala Val Gly Ala Gln Leu Val Ala Leu Arg Ala Ala
                725                 730                 735

Pro Tyr Ala Leu Asn Thr Glu Gln Val Val Ala Ile Ala Ser Ser His
            740                 745                 750

Gly Gly Lys Gln Ala Leu Glu Ala Val Arg Ala Leu Phe Pro Asp Leu
        755                 760                 765

Arg Ala Ala Pro Tyr Ala Leu Ser Thr Ala Gln Leu Val Ala Ile Ala
    770                 775                 780

Ser Asn Pro Gly Gly Lys Gln Ala Leu Glu Ala Val Arg Ala Leu Phe
785                 790                 795                 800

Arg Glu Leu Arg Ala Ala Pro Tyr Ala Leu Ser Thr Glu Gln Val Val
                805                 810                 815

Ala Ile Ala Ser Asn His Gly Gly Lys Gln Ala Leu Glu Ala Val Arg
            820                 825                 830

Ala Leu Phe Arg Gly Leu Arg Ala Ala Pro Tyr Gly Leu Ser Thr Ala
        835                 840                 845

Gln Val Val Ala Ile Ala Ser Ser Asn Gly Gly Lys Gln Ala Leu Glu
    850                 855                 860

Ala Val Trp Ala Leu Leu Pro Val Leu Arg Ala Thr Pro Tyr Asp Leu
865                 870                 875                 880

Asn Thr Ala Gln Ile Val Ala Ile Ala Ser His Asp Gly Gly Lys Pro
                885                 890                 895

Ala Leu Glu Ala Val Trp Ala Lys Leu Pro Val Leu Arg Gly Ala Pro
            900                 905                 910

Tyr Ala Leu Ser Thr Ala Gln Val Val Ala Ile Ala Cys Ile Ser Gly
        915                 920                 925

Gln Gln Ala Leu Glu Ala Ile Glu Ala His Met Pro Thr Leu Arg Gln
    930                 935                 940

Ala Ser His Ser Leu Ser Pro Glu Arg Val Ala Ala Ile Ala Cys Ile
945                 950                 955                 960

Gly Gly Arg Ser Ala Val Glu Ala Val Arg Gln Gly Leu Pro Val Lys
                965                 970                 975

Ala Ile Arg Arg Ile Arg Arg Glu Lys Ala Pro Val Ala Gly Pro Pro
            980                 985                 990

Pro Ala Ser Leu Gly Pro Thr Pro Gln Glu Leu Val Ala Val Leu His
        995                 1000                1005

Phe Phe Arg Ala His Gln Gln Pro Arg Gln Ala Phe Val Asp Ala
    1010                1015                1020

Leu Ala Ala Phe Gln Ala Thr Arg Pro Ala Leu Leu Arg Leu Leu
    1025                1030                1035

Ser Ser Val Gly Val Thr Glu Ile Glu Ala Leu Gly Gly Thr Ile
    1040                1045                1050

Pro Asp Ala Thr Glu Arg Trp Gln Arg Leu Leu Gly Arg Leu Gly
    1055                1060                1065

Phe Arg Pro Ala Thr Gly Ala Ala Ala Pro Ser Pro Asp Ser Leu
    1070                1075                1080

Gln Gly Phe Ala Gln Ser Leu Glu Arg Thr Leu Gly Ser Pro Gly
    1085                1090                1095

Met Ala Gly Gln Ser Ala Cys Ser Pro His Arg Lys Arg Pro Ala
    1100                1105                1110

Glu Thr Ala Ile Ala Pro Arg Ser Ile Arg Arg Ser Pro Asn Asn
    1115                1120                1125

Ala Gly Gln Pro Ser Glu Pro Trp Pro Asp Gln Leu Ala Trp Leu
    1130                1135                1140

Gln Arg Arg Lys Arg Thr Ala Arg Ser His Ile Arg Ala Asp Ser
    1145                1150                1155

Ala Ala Ser Val Pro Ala Asn Leu His Leu Gly Thr Arg Ala Gln
    1160                1165                1170

Phe Thr Pro Asp Arg Leu Arg Ala Glu Pro Gly Pro Ile Met Gln
    1175                1180                1185

Ala His Thr Ser Pro Ala Ser Val Ser Phe Gly Ser His Val Ala
    1190                1195                1200

Phe Glu Pro Gly Leu Pro Asp Pro Gly Thr Pro Thr Ser Ala Asp
    1205                1210                1215

Leu Ala Ser Phe Glu Ala Glu Pro Phe Gly Val Gly Pro Leu Asp
    1220                1225                1230

Phe His Leu Asp Trp Leu Leu Gln Ile Leu Glu Thr
    1235                1240                1245

<210> 2
<211> 502
<212> PRT
<213> Solanum lycopersicum

<400> 2

Met Pro Leu Val Val Arg Phe Pro Asp Val Leu Lys Asn Arg Leu Glu
1               5                   10                  15

Thr Leu Gln Ser Ala Phe Asp Met Ala Ile Asn Ser Gln Gly Tyr Glu
            20                  25                  30

Val His Tyr Gln Gly Val Tyr Pro Val Lys Cys Asn Gln Asp Arg Phe
        35                  40                  45

Val Val Glu Asp Ile Val Lys Phe Gly Ser Pro Tyr Arg Phe Gly Leu
    50                  55                  60

Glu Ala Gly Ser Lys Ser Glu Leu Leu Leu Ala Met Asn Cys Leu Ser
65                  70                  75                  80

Lys Gly Ser Ala Asp Ala Leu Leu Val Cys Asn Gly Phe Lys Asp Thr
                85                  90                  95

Glu Tyr Ile Ser Leu Ala Leu Val Ala Arg Lys Leu Leu Leu Asn Ser
            100                 105                 110

Val Ile Val Leu Glu Gln Glu Glu Glu Leu Asp Leu Val Ile Asp Ile
        115                 120                 125

Ser Arg Lys Met Ser Val Arg Pro Val Ile Gly Leu Arg Ala Lys Leu
    130                 135                 140

Arg Thr Lys His Ser Gly His Phe Gly Ser Thr Ser Gly Glu Lys Gly
145                 150                 155                 160

Lys Phe Gly Leu Thr Thr Thr Gln Ile Leu Arg Val Leu Lys Lys Leu
                165                 170                 175

Asp Glu Ser Gly Met Leu Asp Cys Leu Gln Leu Leu His Phe His Ile
            180                 185                 190

Gly Ser Gln Ile Pro Thr Ser Glu Leu Leu Ala Asp Gly Val Gly Glu
        195                 200                 205

Ala Thr Gln Ile Tyr Ser Glu Leu Val Arg Leu Gly Ala Gly Met Lys
    210                 215                 220

Phe Ile Asp Ile Gly Gly Gly Leu Gly Ile Asp Tyr Asp Gly Ser Lys
225                 230                 235                 240

Ser Ser Asn Ser Asp Val Ser Ala Cys Tyr Ser Ile Glu Lys Asn Ala
                245                 250                 255

Ser Ala Val Val Gln Ala Asp Leu Tyr Val Cys Asp Arg Lys Gly Gly
            260                 265                 270

Lys His Pro Val Ile Cys Ser Glu Ser Gly Arg Ala Ile Val Ser His
        275                 280                 285

His Ser Ile Leu Ile Phe Glu Thr Gly Ser Ala Ser Thr Ser His Val
    290                 295                 300

Ser Thr Gln Pro Ser Ser Gly Gly Leu Gln Ser Leu Val Glu Thr Leu
305                 310                 315                 320

Asn Gly Asp Ala Arg Ala Asp Ser Arg Asn Leu Ser Ala Ala Ala Val
                325                 330                 335

Arg Gly Glu Tyr Asp Thr Cys Leu Ile Tyr Ser Asp Gln Leu Lys Gln
            340                 345                 350

Arg Cys Val Glu Gln Phe Lys Asp Gly Ser Leu Asp Ile Glu Gln Leu
        355                 360                 365

Ala Ala Val Asp Ser Ile Cys Asp Trp Val Ser Lys Ala Ile Gly Val
    370                 375                 380

Ala Asp Pro Val Arg Thr Tyr His Val Asn Leu Ser Val Phe Thr Ser
385                 390                 395                 400

Ile Pro Asp Phe Trp Gly Phe Ser Gln Leu Phe Pro Ile Val Pro Ile
                405                 410                 415

His Arg Leu Asp Glu Lys Pro Thr Met Arg Gly Ile Leu Ser Asp Leu
            420                 425                 430

Thr Cys Asp Ser Asp Gly Lys Val Asp Lys Phe Ile Gly Gly Glu Ser
        435                 440                 445

Ser Leu Pro Leu His Glu Ile Gly Ser Gly Asp Gly Gly Arg Tyr Tyr
    450                 455                 460

Leu Gly Met Phe Leu Gly Gly Ala Tyr Glu Glu Ala Leu Gly Gly Leu
465                 470                 475                 480

His Asn Leu Phe Gly Gly Pro Ser Val Val Arg Val Met Gln Ser Asp
                485                 490                 495

Ser Pro His Ser Phe Ala
            500

<210>  3
<211>  15
<212>  DNA
<213>  ARTIFICIAL

<220>
<223>  ADC Box

<400>  3
gggggwagcc gwggc                                                        15

<210>  4
<211>  15
<212>  DNA
<213>  ARTIFICIAL

<220>
<223>  ADC Box

<400>  4
gggggtagcc ggggc                                                        15

<210>  5
<211>  25
<212>  DNA
<213>  ARTIFICIAL

<220>
<223>  ADC Box

<400>  5

ttttgagggg gtagccgggg ctctg                                                25

<210>  6
<211>  21
<212>  DNA
<213>  ARTIFICIAL

<220>
<223>  ADC Box

<400>  6
ttttgagggg gtggggctct g                                                    21

<210>  7
<211>  25
<212>  DNA
<213>  ARTIFICIAL

<220>
<223>  ADC Box

<400>  7
ttttgagggg gtagccgggg cctcg                                                25

<210>  8
<211>  21
<212>  DNA
<213>  ARTIFICIAL

<220>
<223>  ADC Box

<400>  8
   ttttgagggg agggggcctc g                                                     21

<210>  9
<211>  17
<212>  DNA
<213>  ARTIFICIAL

<220>
<223>  ADC Box

<400>  9
gagggggtag ccggggc                                                         17

<210>  10
<211>  17
<212>  DNA
<213>  ARTIFICIAL

<220>
<223>  ADC Box

<400>  10
gaggaggtag ccgaggc                                                         17

<210>  11
<211>  17
<212>  DNA
<213>  ARTIFICIAL

<220>
<223>  ADC Box

<400>  11
gagggggtag ccgaggc                                                      17


<210>  12
<211>  17
<212>  DNA
<213>  ARTIFICIAL

<220>
<223>  ADC Box

<400>  12
gggggggtag ccggggc                                                      17


<210>  13
<211>  17
<212>  DNA
<213>  ARTIFICIAL

<220>
<223>  ADC Box

<400>  13
   gagggggaag ccggggc                                                       17


<210>  14
<211>  53
<212>  DNA
<213>  ARTIFICIAL

<220>
<223>  ADC Box

<400>  14
cagtccctac agagccgagg ctccggcctc ggcttcggct tttagccagg agc              53


<210>  15
<211>  53
<212>  DNA
<213>  ARTIFICIAL

<220>
<223>  ADC Box

<400>  15
cagtagcgga cgagccgagg ctgcggcctc ggctgctagc caggagcgtg ctg              53


<210>  16
<211>  61
<212>  DNA

<213>  ARTIFICIAL

<220>
<223>  ADC Box

<400>  16
ggcaaaggca aagccggggc ttctgtgcct gaagcctcgg catcctgcga agacagttct      60

t                                                                      61


<210>  17
<211>  53
<212>  DNA
<213>  ARTIFICIAL

<220>
<223>  ADC Box

<400>  17
cggcaaagat cgagtcgagg ctccggcctc ggccgcgccc tgcggccaaa aga             53


<210>  18
<211>  52
<212>  DNA
<213>  ARTIFICIAL

<220>
<223>  ADC Box

<400>  18
gatttgaggg ggagccgagg ctgcggcctc ggcatttttt aaagcccctt tt              52


<210>  19
<211>  53
<212>  DNA
<213>  ARTIFICIAL

<220>
<223>  ADC Box

<400>  19
gattagaggg gaagccgagg ctgcggcctc ggcatttttt aaaagcccct ttg             53


<210>  20
<211>  52
<212>  DNA
<213>  ARTIFICIAL

<220>
<223>  ADC Box

<400>  20
gatttgaggg ggagccgagg ctgcggcctc ggcatttttt ttagccccct tt             52


<210>  21
<211>  53
<212>  DNA
<213>  ARTIFICIAL

<220>
<223>  ADC Box

<400>  21
ttttgagggg gtagccgggg cctcggcctc ggcgggtttt aatagccccc aac          53


<210>  22
<211>  52
<212>  DNA
<213>  ARTIFICIAL

<220>
<223>  ADC Box

<400>  22
ttttgagggg gtagccgggg ctccggcctc ggcgggtttc aaagccccca cc          52


<210>  23
<211>  52
<212>  DNA
<213>  ARTIFICIAL

<220>
<223>  ADC Box

<400>  23
gtttgcgggg gtagccgggg ctccggcctc ggcggctttc taagccccct tc          52


<210>  24
<211>  53
<212>  DNA
<213>  ARTIFICIAL

<220>
<223>  ADC Box

<400>  24
gattagaggg ggtagccgag gctccggcct cggcatcttt caaagccccc ttc          53


<210>  25
<211>  54
<212>  RNA
<213>  ARTIFICIAL

<220>
<223>  ADC Box

<400>  25
cuuuugaggg gguagccggg gcucuggccu cggcggguuc uaaagccccc agcu          54


<210>  26
<211>  24
<212>  DNA
<213>  ARTIFICIAL

<220>
<223>  ADC Box

<400>  26
agagccgagg cccggcccgg cttc                                              24

<210>  27
<211>  43
<212>  DNA
<213>  ARTIFICIAL

<220>
<223>  ADC Box

<400>  27
cgagggggag ccggggctcc ggcccggcgg gaaagccccc acc                         43